# EUROPEAN PATENT APPLICATION

(11) **EP 4 851 129 A1**
(43) Date of publication of application: **09.12.2026**
(21) Application number: 25749058.1
(22) Date of filing: 31.01.2025
(51) Int. Cl.: C07C 47/347, C07C 7/04, C07C 13/61, C07C 29/141, C07C 31/27, C07C 45/50, C07C 47/133

(54) **PRODUCTION METHOD FOR ALDEHYDE, PRODUCTION METHOD FOR ALCOHOL, PRODUCTION METHOD FOR CYCLIC DIENE-CONTAINING COMPOSITION, AND CYCLIC DIENE-CONTAINING COMPOSITION**

(30) Priority: 02.02.2024 JP 2024015094; 08.02.2024 JP 2024018008
(71) Applicant: Mitsubishi Chemical Corporation, Tokyo 100-8251 (JP)
(72) Inventor: TASHIMA, Naoto, Tokyo 100-8251 (JP); SUNAMI, Kazuaki, Tokyo 100-8251 (JP); HINOISHI, Hiroki, Tokyo 100-8251 (JP)
(74) Representative: Hoffmann Eitle
(86) International application number: PCT/JP2025/003232
(87) International publication number: WO 2025/164772

(57) **Abstract**

A method for producing an aldehyde by subjecting a cyclic diene in a cyclic diene-containing composition to a hydroformylation reaction, the method including setting a content proportion of an oxygen-containing compound contained in the cyclic diene-containing composition to a predetermined threshold or less. A method for producing a cyclic diene-containing composition containing a cyclic diene by thermal decomposition of a hydrocarbon-containing composition to produce a hydrocarbon decomposition product, and distillation purification of the hydrocarbon decomposition product, the method including setting a content proportion of an oxygen-containing compound contained in the cyclic diene-containing composition to a predetermined threshold or less.

## Description

### Technical Field

The present invention relates to a method for producing an aldehyde, a method for producing an alcohol, a method for producing a cyclic diene-containing composition, and a cyclic diene-containing composition.

### Background Art

Aldehydes such as alicyclic aldehydes are useful as raw materials for additives of plasticizers, adhesives, microbicides, and the like. Alcohols such as alicyclic alcohols obtained by subjecting aldehydes such as alicyclic aldehydes to a hydrogenation reaction are useful as raw material monomers directed to achieve higher heat resistance, flex resistance, or lower retardation of polyurethane-based resins, polyester-based resins, or polycarbonate-based resins. Specifically, alicyclic aldehydes such as tricyclodecane dicarbaldehyde and alicyclic alcohols such as tricyclodecane dimethanol and pentacyclopentadecane dimethanol have attracted attention from the viewpoint of achieving higher functionality in various uses and excellent industrial productivity.

As a method for producing an alicyclic aldehyde such as tricyclodecane dicarbaldehyde, there has been known a method including subjecting a hydrocarbon-containing composition such as naphtha, coal, and natural gas to thermal decomposition, thereby producing a C5 hydrocarbon fraction, heating the C5 hydrocarbon fraction and thereby subjecting a cyclic diene (also referred to as "cyclic diene" in the present invention) such as cyclopentadiene in the C5 hydrocarbon fraction to a dimerization reaction to form a cyclic diene such as dicyclopentadiene corresponding to the cyclic diene, then purifying the C5 hydrocarbon fraction after the dimerization reaction and providing a composition containing a high concentration of the cyclic diene such as dicyclopentadiene (hereinafter, referred to as "cyclic diene-containing composition"), and subjecting the obtained cyclic diene-containing composition to a hydroformylation reaction in the presence of a Long-Form Periodic Table Group 8 to 10 metal (hereinafter sometimes simply referred to as "Group 8 to 10 metal")-organophosphorus-based complex catalyst to convert a reactant from the hydroformylation reaction into an alicyclic aldehyde corresponding to the cyclic diene.

The catalyst used in the hydroformylation reaction of the cyclic diene-containing composition contains an expensive Group 8 to 10 metal such as rhodium. Therefore, when a factor inhibiting the hydroformylation reaction is present in the reaction system, the amount of the catalyst used must be increased, resulting in an increase in production cost. Therefore, it is ideal to remove the factor inhibiting the hydroformylation reaction as much as possible.

As the method for providing the alicyclic aldehyde by feeding the cyclic diene-containing composition to a hydroformylation reaction as described above, for example, an Experimental Example of Patent Literature 1 describes a technique for producing bisformyltricyclodecane by hydroformylation of highly purified dicyclopentadiene.

Patent Literature 2 describes a technique for reducing conjugated diene, focusing on the conjugated diene contained in dicyclopentadiene as an impurity that inhibits a hydroformylation reaction.

However, the techniques described in Patent Literatures 1 and 2 have problems, such as complication of production process, increased investment in equipment and production cost, and increased utility cost, due to separation and recovery of a highly pure cyclic diene by extremely removing impurities contained in a cyclic diene-containing composition.

Even if the impurities contained in the cyclic diene-containing composition are reduced in view of economic efficiency, it has not been fully elucidated what impurities affect the activity of the hydroformylation reaction catalyst, and what impurities affect the recovery rate of expensive rhodium metal when the hydroformylation reaction catalyst is recovered from a process liquid after the hydroformylation reaction and reused.

### Citation List

### Patent Literature

Patent Literature 1: JP2005-139181A
Patent Literature 2: JPH11-80067A

### Summary of Invention

### Technical Problem

An object of the present invention is to solve these problems.

That is, the present invention provides a method for producing an aldehyde such as tricyclodecane dicarbaldehyde corresponding to a cyclic diene such as dicyclopentadiene using a cyclic diene-containing composition containing the cyclic diene, the method being capable of efficiently producing the aldehyde while suppressing a decrease in catalytic activity of a hydroformylation reaction catalyst, and recovering an expensive rhodium metal from a process liquid after a hydroformylation reaction at a high recovery rate.

The present invention also provides a cyclic diene-containing composition containing a cyclic diene such as dicyclopentadiene, the composition being used as a starting material for an aldehyde such as tricyclodecane dicarbaldehyde, wherein the composition is capable of efficiently producing the aldehyde while suppressing a decrease in catalytic activity of a hydroformylation reaction catalyst, and recovering an expensive rhodium metal from a process liquid after a hydroformylation reaction at a high recovery rate; and a method for producing the cyclic diene-containing composition.

Furthermore, the present invention provides a method for producing an alcohol, wherein an aldehyde is produced by the method for producing the aldehyde, and an alcohol is produced from the aldehyde.

### Solution to Problem

As a result of diligent research to solve the problems described above, the present inventor has found that the problems described above can be solved by setting a content proportion of an oxygen-containing compound contained in a cyclic diene-containing composition to a predetermined value or less.

That is, the gist of the present invention is as follows.

[1] A method for producing an aldehyde by subjecting a cyclic diene in a cyclic diene-containing composition to a hydroformylation reaction,
   the method including setting a content proportion of an oxygen-containing compound contained in the cyclic diene-containing composition to a predetermined threshold or less.
[2] The method for producing an aldehyde according to [1], wherein the oxygen-containing compound is a cyclic diene oxide, and the cyclic diene excludes the cyclic diene oxide.
[3] The method for producing an aldehyde according to [2], wherein a content proportion of the cyclic diene oxide contained in the cyclic diene-containing composition is 1.2 GC area% or less.
[4] The method for producing an aldehyde according to [2] or [3], wherein a content proportion of the cyclic diene contained in the cyclic diene-containing composition is 60 GC area% or more.
[5] The method for producing an aldehyde according to [3] or [4], wherein the content proportion of the cyclic diene oxide contained in the cyclic diene-containing composition is 0.5 GC area% or less, preferably 0.35 GC area% or less, more preferably 0.25 GC area% or less, further preferably 0.14 GC area% or less, and particularly preferably 0.05 GC area% or less.
[6] The method for producing an aldehyde according to any of [2] to [5], wherein a content proportion of the cyclic diene oxide is measured by measurement method 1 described below:
   measurement method 1
      measuring a total content proportion of peaks for an elution time from 17.7 minutes to 18.8 minutes by analysis under GC measurement conditions described below:
   GC measurement conditions
      GC apparatus: gas chromatogram measuring apparatus (trade name: GC-2025, available from Shimadzu Corporation)
      detector: hydrogen flame ionization detector (FID)
      carrier gas: helium (column flow rate: 1.65 mL/min)
      column: capillary column (product name: DB-1, available from Agilent Technologies; size: length 30 m × inner diameter 0.25 mm, film thickness 1.00 µm)
      column temperature: 50°C (retention time: 5 minutes) → temperature increase at 10°C/min → 300°C (retention time: none)
      inlet temperature: 200°C
      detector temperature: 300°C
      sample amount: 0.3 µL (split ratio: 1/30).
[7] The method for producing an aldehyde according to any of [2] to [6], wherein the cyclic diene is a polycyclic diene.
[8] The method for producing an aldehyde according to [7], wherein the cyclic diene is dicyclopentadiene.
[9] The method for producing an aldehyde according to any of [2] to [8], wherein the cyclic diene oxide is a compound having any skeleton selected from a tricyclodecene skeleton and a tricyclodecadiene skeleton, and having an oxygen-containing functional group.
[10] The method for producing an aldehyde according to any of [2] to [9], wherein the cyclic diene oxide contains at least one selected from the group consisting of a cyclic diene having a carbonyl group, a cyclic diene having a hydroxyl group, and a cyclic monoene having an epoxy group, which correspond to the cyclic diene.
[11] The method for producing an aldehyde according to any of [8] to [10], wherein
   the cyclic diene is dicyclopentadiene, and
   the cyclic diene oxide contains at least one selected from the group consisting of a compound in which one enantiomer is represented by General Formula (I) described below, a compound in which one enantiomer is represented by General Formula (II) described below, and a compound in which one enantiomer is represented by General Formula (III) described below: 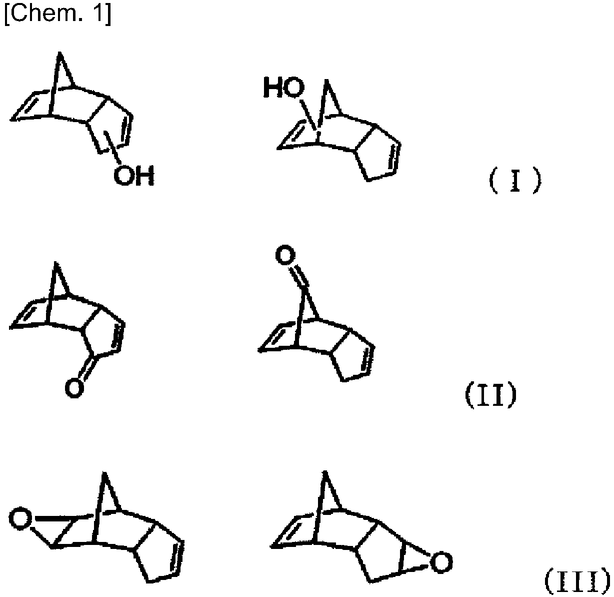
   where, in General Formula (I), a hydroxyl group (-OH) is bonded to any position in an illustrated dicyclopentadiene ring structure.
[12] The method for producing an aldehyde according to [11], wherein the cyclic diene is dicyclopentadiene, and the aldehyde is tricyclodecane dicarbaldehyde.
[13] The method for producing an aldehyde according to any of [2] to [12], wherein
   the cyclic diene-containing composition is a composition obtained by thermal decomposition of a hydrocarbon-containing composition to produce a hydrocarbon decomposition product, and distillation purification of the hydrocarbon decomposition product, and
   the method includes performing the distillation purification so that a content proportion of the cyclic diene oxide contained in the cyclic diene-containing composition is a predetermined threshold or less.
[14] The method for producing an aldehyde according to any of [2] to [13], the method including controlling a storage condition of the cyclic diene-containing composition so that a content proportion of the cyclic diene oxide contained in the cyclic diene-containing composition is a predetermined threshold or less.
[15] The method for producing an aldehyde according to [14], wherein the controlling the storage condition includes controlling a dissolved oxygen concentration in the cyclic diene-containing composition.
[16] The method for producing an aldehyde according to [14], wherein the controlling the storage condition includes adding an antioxidant to the cyclic diene-containing composition.
[17] A method for producing an alcohol, the method including producing an aldehyde by the method described in any of [1] to [16], and producing an alcohol from the aldehyde.
[18] The method for producing an alcohol according to [17], wherein the cyclic diene is dicyclopentadiene, the aldehyde is tricyclodecane dicarbaldehyde, and the alcohol is tricyclo[5.2.1.0^{2,6}]decanedimethanol.
[19] The method for producing an aldehyde according to [1], wherein the oxygen-containing compound is a peroxide.
[20] The method for producing an aldehyde according to [19], wherein a concentration of the peroxide contained in the cyclic diene-containing composition is 2.8 mmol/L or less.
[21] The method for producing an aldehyde according to [20], wherein the concentration of the peroxide contained in the cyclic diene-containing composition is 2.0 mmol/L or less, preferably 1.0 mmol/L or less, more preferably 0.6 mmol/L or less, and particularly preferably 0.3 mmol/L or less.
[22] The method for producing an aldehyde according to any of [19] to [21], wherein the peroxide contains a peroxide of an alkene compound.
[23] The method for producing an aldehyde according to any of [19] to [22], wherein the concentration of the peroxide is measured by measurement method 1 described below:
   measurement method 1
   adding a mixed liquid of chloroform and acetic acid to the cyclic diene-containing composition under a nitrogen atmosphere, and further adding a saturated aqueous potassium iodide solution, followed by stirring and dilution with distilled water to prepare a measurement sample; and subjecting the prepared measurement sample to iodometric titration using a 0.01 mol/L aqueous sodium thiosulfate solution as a standard liquid, and determining the concentration (mmol/L) of the peroxide in the cyclic diene-containing composition based on an amount of the standard liquid consumed.
[24] The method for producing an aldehyde according to any of [19] to [23], wherein the cyclic diene is a polycyclic diene.
[25] The method for producing an aldehyde according to [24], wherein the polycyclic diene is dicyclopentadiene.
[26] The method for producing an aldehyde according to any of [19] to [25], wherein
   the cyclic diene-containing composition is a composition obtained by thermal decomposition of a hydrocarbon-containing composition to produce a hydrocarbon decomposition product, and distillation purification of the hydrocarbon decomposition product, and
   the method includes performing the distillation purification so that a concentration of the peroxide contained in the cyclic diene-containing composition is a predetermined threshold or less.
[27] The method for producing an aldehyde according to [26], wherein the hydrocarbon decomposition product contains an antioxidant.
[28] The method for producing an aldehyde according to [26] or [27], the method including adding an antioxidant to the hydrocarbon decomposition product so that the concentration of the peroxide contained in the cyclic diene-containing composition is a predetermined threshold or less.
[29] The method for producing an aldehyde according to any of [26] to [28], the method including controlling a storage condition of the cyclic diene-containing composition so that the concentration of the peroxide contained in the cyclic diene-containing composition is a predetermined threshold or less.
[30] The method for producing an aldehyde according to [29], wherein the controlling the storage condition includes controlling a dissolved oxygen concentration in the cyclic diene-containing composition.
[31] The method for producing an aldehyde according to [29], wherein the controlling the storage condition includes adding an antioxidant to the cyclic diene-containing composition.
[32] The method for producing an aldehyde according to any of [19] to [31], wherein the cyclic diene is dicyclopentadiene, and the aldehyde is tricyclodecane dicarbaldehyde.
[33] A method for producing an alcohol, the method including producing an aldehyde by the method described in any of [19] to [32], and producing a corresponding alcohol from the produced aldehyde.
[34] The method for producing an alcohol according to [33], wherein the cyclic diene is dicyclopentadiene, the aldehyde is tricyclodecane dicarbaldehyde, and the alcohol is tricyclo[5.2.1.0^{2,6}]decanedimethanol.
[35] A method for producing a cyclic diene-containing composition containing a cyclic diene by thermal decomposition of a hydrocarbon-containing composition to produce a hydrocarbon decomposition product, and distillation purification of the hydrocarbon decomposition product,
   the method including setting a content proportion of an oxygen-containing compound contained in the cyclic diene-containing composition to a predetermined threshold or less.
[36] The method for producing a cyclic diene-containing composition according to [35], wherein the oxygen-containing compound is a cyclic diene oxide, and the cyclic diene excludes the cyclic diene oxide.
[37] The method for producing a cyclic diene-containing composition according to [36], wherein a content proportion of the cyclic diene oxide contained in the cyclic diene-containing composition is 1.2 GC area% or less.
[38] The method for producing a cyclic diene-containing composition according to [36] or [37], the method including performing the distillation purification so that the content proportion of the cyclic diene oxide contained in the cyclic diene-containing composition is a predetermined threshold or less.
[39] The method for producing a cyclic diene-containing composition according to any of [36] to [38], the method including controlling a storage condition of the cyclic diene-containing composition so that the content proportion of the cyclic diene oxide contained in the cyclic diene-containing composition is a predetermined threshold or less.
[40] The method for producing a cyclic diene-containing composition according to [39], wherein the controlling the storage condition includes controlling a dissolved oxygen concentration in the cyclic diene-containing composition.
[41] The method for producing a cyclic diene-containing composition according to [39], wherein the controlling the storage condition includes adding an antioxidant to the cyclic diene-containing composition.
[42] The method for producing a cyclic diene-containing composition according to any of [36] to [41], wherein a content proportion of the cyclic diene contained in the cyclic diene-containing composition is 60 GC area% or more.
[43] The method for producing a cyclic diene-containing composition according to [41] or [42], wherein a content proportion of the cyclic diene oxide contained in the cyclic diene-containing composition is 0.5 GC area% or less, preferably 0.35 GC area% or less, more preferably 0.25 GC area% or less, further preferably 0.14 GC area% or less, and particularly preferably 0.05 GC area% or less.
[44] The method for producing a cyclic diene-containing composition according to any of [36] to [43], wherein a content proportion of the cyclic diene oxide is measured by measurement method 1 described below:
   measurement method 1
      measuring a total content proportion of peaks for an elution time from 17.7 minutes to 18.8 minutes by analysis under GC measurement conditions described below.
   GC measurement conditions
      GC apparatus: gas chromatogram measuring apparatus (trade name: GC-2025, available from Shimadzu Corporation)
      detector: hydrogen flame ionization detector (FID)
      carrier gas: helium (column flow rate: 1.65 mL/min)
      column: capillary column (product name: DB-1, available from Agilent Technologies; size: length 30 m × inner diameter 0.25 mm, film thickness 1.00 µm)
      column temperature: 50°C (retention time: 5 minutes) → temperature increase at 10°C/min → 300°C (retention time: none)
      inlet temperature: 200°C
      detector temperature: 300°C
      sample amount: 0.3 µL (split ratio: 1/30).
[45] The method for producing a cyclic diene-containing composition according to any of [36] to [44], wherein the cyclic diene is a polycyclic diene.
[46] The method for producing a cyclic diene-containing composition according to [45], wherein the cyclic diene is dicyclopentadiene.
[47] The method for producing a cyclic diene-containing composition according to any of [36] to [46], wherein the cyclic diene oxide is a compound having any skeleton selected from a tricyclodecene skeleton and a tricyclodecadiene skeleton, and having an oxygen-containing functional group.
[48] The method for producing a cyclic diene-containing composition according to any of [36] to [47], wherein the cyclic diene oxide contains at least one selected from the group consisting of a cyclic diene having a carbonyl group, a cyclic diene having a hydroxyl group, and a cyclic monoene having an epoxy group.
[49] The method for producing a cyclic diene-containing composition according to any of [46] to [48], wherein
   the cyclic diene is dicyclopentadiene, and
   the cyclic diene oxide contains at least one selected from the group consisting of a compound in which one enantiomer is represented by General Formula (I) described below, a compound in which one enantiomer is represented by General Formula (II) described below, and a compound in which one enantiomer is represented by General Formula (III) described below: 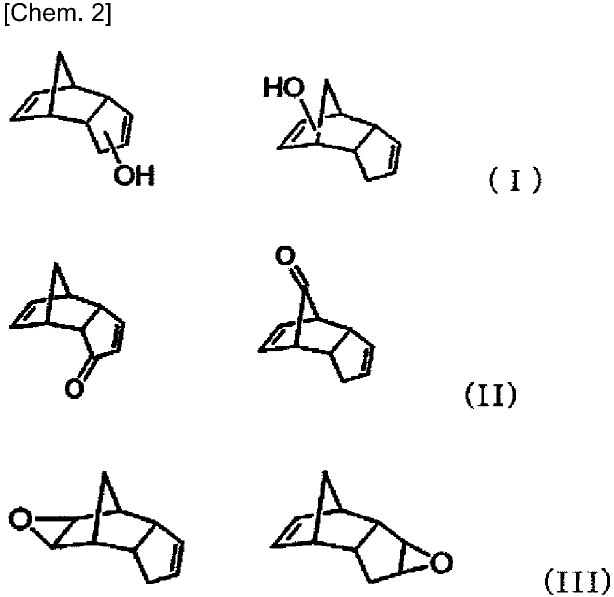
   where, in General Formula (I), a hydroxyl group (-OH) is bonded to any position in an illustrated dicyclopentadiene ring structure.
[50] The method for producing a cyclic diene-containing composition according to any of [36] to [49], wherein the hydrocarbon-containing composition is naphtha.
[51] The method for producing a cyclic diene-containing composition according to [35], wherein the oxygen-containing compound is a peroxide.
[52] The method for producing a cyclic diene-containing composition according to [51], the method including performing the distillation purification so that a concentration of the peroxide contained in the cyclic diene-containing composition is a predetermined threshold or less.
[53] The method for producing a cyclic diene-containing composition according to [51] or [52], wherein the hydrocarbon decomposition product contains an antioxidant.
[54] The method for producing a cyclic diene-containing composition according to any of [51] to [53], the method including adding an antioxidant to the hydrocarbon decomposition product so that the concentration of the peroxide contained in the cyclic diene-containing composition is a predetermined threshold or less.
[55] The method for producing a cyclic diene-containing composition according to any of [51] to [54], the method including controlling a storage condition of the cyclic diene-containing composition so that the concentration of the peroxide contained in the cyclic diene-containing composition is a predetermined threshold or less.
[56] The method for producing a cyclic diene-containing composition according to [55], wherein the controlling the storage condition includes controlling a dissolved oxygen concentration in the cyclic diene-containing composition.
[57] The method for producing a cyclic diene-containing composition according to [55], wherein the controlling the storage condition includes adding an antioxidant to the cyclic diene-containing composition.
[58] The method for producing a cyclic diene-containing composition according to any of [51] to [57], wherein the concentration of the peroxide contained in the cyclic diene-containing composition is 2.8 mmol/L or less.
[59] The method for producing a cyclic diene-containing composition according to any of [51] to [57], wherein a content proportion of the cyclic diene contained in the cyclic diene-containing composition is 60.0 GC area% or more.
[60] The method for producing a cyclic diene-containing composition according to [58] or [59], wherein the concentration of the peroxide contained in the cyclic diene-containing composition is 2.0 mmol/L or less, preferably 1.0 mmol/L or less, more preferably 0.6 mmol/L or less, and particularly preferably 0.3 mmol/L or less.
[61] The method for producing a cyclic diene-containing composition according to any of [51] to [60], wherein the concentration of the peroxide is measured by measurement method 1 described below:
   measurement method 1
   adding a mixed liquid of chloroform and acetic acid to the cyclic diene-containing composition under a nitrogen atmosphere, and further adding a saturated aqueous potassium iodide solution, followed by stirring and dilution with distilled water to prepare a measurement sample; and subjecting the prepared measurement sample to iodometric titration using a 0.01 mol/L aqueous sodium thiosulfate solution as a standard liquid, and determining the concentration (mmol/L) of the peroxide in the cyclic diene-containing composition based on an amount of the standard liquid consumed.
[62] The method for producing a cyclic diene-containing composition according to any of [51] to [61], wherein the cyclic diene is a polycyclic diene.
[63] The method for producing a cyclic diene-containing composition according to [62], wherein the polycyclic diene is dicyclopentadiene.
[64] The method for producing a cyclic diene-containing composition according to any of [51] to [63], wherein the hydrocarbon-containing composition is naphtha.
[65] A cyclic diene-containing composition containing a cyclic diene, wherein
   a content proportion of a cyclic diene oxide in the cyclic diene-containing composition is 1.2 GC area% or less, and
   the cyclic diene excludes the cyclic diene oxide.
[66] The cyclic diene-containing composition according to [65], wherein a content proportion of the cyclic diene is 60 GC area% or more.
[67] The cyclic diene-containing composition according to [65] or [66], wherein the content proportion of the cyclic diene oxide is 0.5 GC area% or less, preferably 0.35 GC area% or less, more preferably 0.25 GC area% or less, further preferably 0.14 GC area% or less, and particularly preferably 0.05 GC area% or less.
[68] The cyclic diene-containing composition according to any of [65] to [67], wherein the content proportion of the cyclic diene oxide is measured by measurement method 1 described below:
   measurement method 1
      measuring a total content proportion of peaks for an elution time from 17.7 minutes to 18.8 minutes by analysis under GC measurement conditions described below:
   GC measurement conditions
      GC apparatus: gas chromatogram measuring apparatus (trade name: GC-2025, available from Shimadzu Corporation)
      detector: hydrogen flame ionization detector (FID)
      carrier gas: helium (column flow rate: 1.65 mL/min)
      column: capillary column (product name: DB-1, available from Agilent Technologies; size: length 30 m × inner diameter 0.25 mm, film thickness 1.00 µm)
      column temperature: 50°C (retention time: 5 minutes) → temperature increase at 10°C/min → 300°C (retention time: none)
      inlet temperature: 200°C
      detector temperature: 300°C
      sample amount: 0.3 µL (split ratio: 1/30).
[69] The cyclic diene-containing composition according to any of [65] to [68], wherein the cyclic diene is a polycyclic diene.
[70] The cyclic diene-containing composition according to [69], wherein the cyclic diene is dicyclopentadiene.
[71] The cyclic diene-containing composition according to any of [65] to [70], wherein the cyclic diene oxide is a compound having any skeleton selected from a tricyclodecene skeleton and a tricyclodecadiene skeleton, and having an oxygen-containing functional group.
[72] The cyclic diene-containing composition according to any of [65] to [71], wherein the cyclic diene oxide contains at least one selected from the group consisting of a cyclic diene having a carbonyl group, a cyclic diene having a hydroxyl group, and a cyclic monoene having an epoxy group.
[73] The cyclic diene-containing composition according to any of [70] to [72], wherein
   the cyclic diene is dicyclopentadiene, and
   the cyclic diene oxide contains at least one selected from the group consisting of a compound in which one enantiomer is represented by General Formula (I) described below, a compound in which one enantiomer is represented by General Formula (II) described below, and a compound in which one enantiomer is represented by General Formula (III) described below: 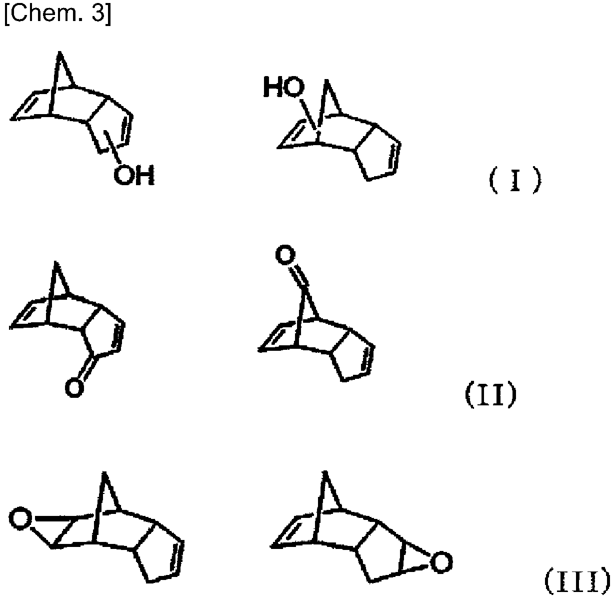
   where, in General Formula (I), a hydroxyl group (-OH) is bonded to any position in an illustrated dicyclopentadiene ring structure.
[74] The cyclic diene-containing composition according to any of [65] to [73], wherein a concentration of the peroxide in the cyclic diene-containing composition is 2.8 mmol/L or less.
[75] The cyclic diene-containing composition according to [74], wherein the concentration of the peroxide is 2.0 mmol/L or less, preferably 1.0 mmol/L or less, more preferably 0.6 mmol/L or less, and particularly preferably 0.3 mmol/L or less.
[76] The cyclic diene-containing composition according to [74] or [75], wherein the peroxide contains a peroxide of an alkene compound.
[77] A method for producing an aldehyde by subjecting the cyclic diene-containing composition described in any of [65] to [76] to a hydroformylation reaction.
[78] A method for producing an alcohol, wherein an aldehyde is produced by the method described in [77], and an alcohol is produced from the aldehyde.

### Advantageous Effects of Invention

According to the present invention, it is possible to provide a method for producing an aldehyde such as tricyclodecane dicarbaldehyde corresponding to a cyclic diene such as dicyclopentadiene using a cyclic diene-containing composition containing the cyclic diene, the method being capable of efficiently producing the aldehyde while suppressing a decrease in catalytic activity of a hydroformylation reaction catalyst, and recovering an expensive rhodium metal from a process liquid after a hydroformylation reaction at a high recovery rate.

In addition, a corresponding alcohol can be efficiently produced from the aldehyde produced by the cyclic diene-containing composition of the present invention.

Further, according to the present invention, it is possible to provide a cyclic diene-containing composition used as a starting material for an aldehyde such as an alicyclic aldehyde, wherein the aldehyde can be efficiently produced from the cyclic diene-containing composition while suppressing a decrease in catalytic activity of a hydroformylation reaction catalyst, and an expensive rhodium metal can be recovered from a process liquid after a hydroformylation reaction at a high recovery rate.

### Brief Description of Drawings

FIG. 1 is a schematic system diagram illustrating an embodiment of a production process configured to separate and recover a cyclic diene-containing composition of the present invention from a C5 hydrocarbon fraction, according to the present invention.
FIG. 2 is a graph illustrating a relationship between a content proportion (GC area%) of a cyclic diene oxide (DCPD oxide) and a reaction rate constant (k2) in Experimental Examples 1 to 7.
FIG. 3 is a graph illustrating a relationship between the content proportion (GC area%) of the cyclic diene oxide (DCPD oxide) and a rate of decrease in the reaction rate constant (k2) in Experimental Examples 1 to 7.
FIG. 4 is a graph illustrating a relationship between the content proportion (GC area%) of the cyclic diene oxide (DCPD oxide) and a rhodium (Rh) recovery rate in Experimental Examples 1 to 7.
FIG. 5 is a diagram illustrating a gas chromatogram of a DCPD-containing composition produced in Reference Experimental Example 2.

### Description of Embodiments

Hereinafter, the present invention will be described in detail. However, the present invention is not limited to the following description and can be carried out with optional modifications within a scope that does not depart from the gist of the present invention.

Unless otherwise noted, a numerical range represented by "to" in the present specification means a range including the numerical values written before and after "to" as the lower limit value and the upper limit value respectively, and "A to B" means "A or more and B or less".

In the present specification, "including A or B" means "including A", "including B", and "including A and B" unless otherwise specified.

In the present specification, the term "GC area%" refers to a compositional proportion of each component measured by using a gas chromatograph (GC) and a method using total area in gas chromatography and calculated as an area content (unit: GC area%) of each peak component when the total area of GC peaks of all products in the gas chromatogram is taken as 100%. The details of the GC measurement conditions will be described in the experimental examples described below.

In the present specification, the term "mass%" refers to a content proportion of a predetermined component contained in 100 mass% of a total amount, and the term "wt.%" refers to a content proportion of a predetermined component contained in 100 wt.% of a total amount.

The terms "mass%" and "wt%" are synonymous with each other.

The term "optional/any" or "freely" means that a subsequently described circumstance may or may not occur, and therefore the description includes cases where the circumstance occurs and cases where it does not occur.

The term "about" as used herein may include a range from -20% to +20% of the stated value. For example, approximately 75°C includes a range of 60°C to 90°C.

All steps described herein can be carried out in any suitable order unless otherwise specified herein or otherwise clearly contradicted by context.

In the present specification, "hydrocarbon" means naphtha, coal, and natural gas. More specifically, the "hydrocarbon" means hydrocarbons including, for example, fossil fuels and refined petroleum products, such as naphtha, heavy aromatic naphtha, crude oil, gasoline, kerosene, diesel oil, light oil, heavy oil, extra heavy oil, Class A heavy oil, Class B heavy oil, Class C heavy oil, jet fuel oil, tar, GTL (gas-to-liquid) oil, CTL (coal-to-liquid) oil, coal, coke, natural gas, LNG (liquefied natural gas), LPG (liquefied petroleum gas), sour gas, oilfield gas, and oilfield concentrates.

In the present specification, the "method for producing an aldehyde of the present invention", the "method for producing an alcohol of the present invention", the "method for producing a cyclic diene-containing composition of the present invention", and the "cyclic diene-containing composition of the present invention" are collectively referred to as "the present invention".

Firstly, in the present invention, the "oxygen-containing compound" (hereinafter, may be referred to as "oxygen-containing compound of the present invention") whose content proportion in the cyclic diene-containing composition is set to a predetermined threshold or less and the cyclic diene contained in the cyclic diene-containing composition will be described.

The cyclic diene-containing composition, i.e., a first embodiment of the present invention, the method for producing an aldehyde, i.e., a second embodiment of the present invention, the method for producing an alcohol, i.e., a third embodiment of the present invention, and the method for producing a cyclic diene-containing composition, i.e., a fourth embodiment of the present invention (hereinafter, the inventions of the first to fourth embodiments may be collectively referred to as "the present invention") will be described below.

### Oxygen-Containing Compound

The oxygen-containing compound of the present invention is not particularly limited as long as the effects of the present invention can be exhibited by setting the content proportion thereof in the cyclic diene-containing composition to a predetermined value or less. Specific examples of the oxygen-containing compound include a cyclic diene oxide described below and a peroxide described below, because the effects of the present invention can be more remarkably achieved. The oxygen-containing compound of the present invention may be only one of these compounds, or may be two or more of these compounds. Details of the cyclic diene oxide and the peroxide will be described below.

In the present invention, the "cyclic diene oxide" is not included in the cyclic diene in the cyclic diene-containing composition of the present invention, and the cyclic diene according to the present invention excludes the cyclic diene oxide.

By setting the content proportion of the cyclic diene oxide in the cyclic diene-containing composition, as the oxygen-containing compound of the present invention, to a predetermined threshold or less, it is possible to efficiently produce an alicyclic aldehyde from the cyclic diene-containing composition while suppressing a decrease in catalytic activity of a hydroformylation reaction catalyst, and to recover an expensive rhodium metal from a process liquid after a hydroformylation reaction at a high recovery rate, in the production of the aldehyde of the present invention.

By setting the concentration of the peroxide in the cyclic diene-containing composition, as the oxygen-containing compound of the present invention, to a predetermined threshold or less, it is possible to efficiently produce an alicyclic aldehyde while suppressing a decrease in catalytic activity of a hydroformylation reaction catalyst, even when a procedure including recovery from a reaction liquid after a hydroformylation reaction and reuse is repeated in the production of the aldehyde of the present invention.

### Cyclic Diene

The cyclic diene (also referred to as "cyclic diene") according to the present invention is a component constituting the cyclic diene-containing composition of the present invention.

The "cyclic diene" in the method for producing an aldehyde of the present invention described below and the method for producing a cyclic diene-containing composition of the present invention described below can be treated as having the same meaning as the cyclic diene in the cyclic diene-containing composition of the present invention.

The cyclic diene in the present invention is not particularly limited as long as the cyclic diene is a compound having a ring structure in the molecule and having two nonconjugated carbon-carbon double bonds in the ring structure, but is preferably a compound that can be used as a starting substance for producing an alicyclic alcohol.

One embodiment of the cyclic diene is a polycyclic diene having two or more ring structures in the molecule wherein the two or more ring structures each have a nonconjugated carbon-carbon double bond.

Alternatively, another embodiment of the cyclic diene is a compound produced by subjecting a cyclic diene having one ring structure to dimerization.

Specific examples of the cyclic diene include dicyclopentadiene, 1,4-cyclohexadiene, methylcyclopentadiene, methyldicyclopentadiene, and methyltetrahydroindene.

Among these cyclic dienes, dicyclopentadiene is preferred from the viewpoints of industrial availability, excellent hydroformylation reactivity, and excellent production efficiency of the alicyclic alcohol.

One type of these cyclic dienes can be used alone, or a combination of two or more types of these cyclic dienes can be used.

Hereinafter, firstly, the cyclic diene-containing composition and the method for producing a cyclic diene-containing composition of the present invention will be described, and then the method for producing an aldehyde and the method for producing an alcohol of the present invention will be described.

### Cyclic Diene-Containing Composition

The cyclic diene-containing composition of the present invention is a composition containing the cyclic diene described above, in which the content proportion of a cyclic diene oxide in the cyclic diene-containing composition is 1.2 GC area% or less. However, as described above, the cyclic diene does not include the cyclic diene oxide.

In the present invention, the lower limit of the content proportion (unit: GC area%) of the cyclic diene in the cyclic diene-containing composition is not particularly limited. The lower limit can be 60.0 GC area% or more, and is preferably 63.0 GC area% or more, more preferably 66.0 GC area% or more, further preferably 70.0 GC area% or more, particularly preferably 75.0 GC area% or more, and most preferably 80.0 GC area% or more, relative to 100% of the total GC area of the cyclic diene-containing composition, from the viewpoint of producing an aldehyde such as an alicyclic aldehyde in a short reaction time and in a high yield using the cyclic diene-containing composition as a starting material.

Meanwhile, in the present invention, the upper limit of the content proportion (unit: GC area%) of the cyclic diene in the cyclic diene-containing composition is not particularly limited. The upper limit can be 99.95 GC area% or less, and is preferably 99.9 GC area% or less, more preferably 97.5 GC area% or less, further preferably 95.0 GC area% or less, particularly preferably 90.0 GC area% or less, and most preferably 85.0 GC area% or less, relative to 100% of the total GC area of the cyclic diene-containing composition, from the viewpoint of economic efficiency such as production cost required for purification and separation of the cyclic diene-containing composition, and from the viewpoint of having fluidity at normal temperature and excellent handleability.

The above upper and lower limits can be freely combined. For example, although the content proportion (unit: GC area%) of the cyclic diene in the cyclic diene-containing composition in the present invention is not particularly limited, the content proportion can be 60.0 GC area% or more and 99.95 GC area% or less, and is preferably 63.0 GC area% or more and 99.9 GC area% or less, more preferably 66.0 GC area% or more and 97.5 GC area% or less, further preferably 70.0 GC area% or more and 95.0 GC area% or less, particularly preferably 75.0 GC area% or more and 90.0 GC area% or less, and most preferably 80.0 GC area% or more and 85.0 GC area% or less, relative to 100% of the total GC area of the cyclic diene-containing composition.

In the cyclic diene-containing composition and the method for producing the same of the present invention, and the method for producing an aldehyde of the present invention, the lower limit of the GC area ratio of the content proportion (unit: GC area%) of the cyclic diene oxide to the content proportion (unit: GC area%) of the cyclic diene is not particularly limited. The lower limit can be 0.00001 or more, and is preferably 0.00002 or more, more preferably 0.00004 or more, further preferably 0.00006 or more, and particularly preferably 0.00008 or more, from the viewpoint of economic efficiency such as production cost required for purification and separation of the cyclic diene-containing composition.

Meanwhile, in the present invention, the upper limit of the GC area ratio can be 0.040 or less, and is preferably 0.015 or less, more preferably 0.0035 or less, further preferably 0.0025 or less, and particularly preferably 0.0015 or less, from the viewpoint of suppressing a decrease in catalytic activity of the hydroformylation reaction catalyst and recovering expensive rhodium metal at a high recovery rate.

The above upper and lower limits can be freely combined. For example, although the GC area ratio of the content proportion (unit: GC area%) of the cyclic diene oxide to the content proportion (unit: GC area%) of the cyclic diene in the present invention is not particularly limited, the GC area ratio can be 0.00001 or more and 0.040 or less, and is preferably 0.00002 or more and 0.015 or less, more preferably 0.00004 or more and 0.0035 or less, further preferably 0.00006 or more and 0.0025 or less, and particularly preferably 0.00008 or more and 0.0015 or less.

A method for measuring the content proportion of the cyclic diene in the cyclic diene-containing composition will be described in detail below.

Examples of the method for controlling the content proportion of the cyclic diene in the cyclic diene-containing composition include methods of controlling the amount of the cyclic diene by an extraction method, a purification condition, or a distillation condition, and a method of combining these methods.

### Cyclic Diene Oxide

In the cyclic diene-containing composition and the method for producing the same of the present invention, and the method for producing an aldehyde of the present invention, in a case where the oxygen-containing compound is a cyclic diene oxide, by setting the content proportion of the cyclic diene oxide contained in the cyclic diene-containing composition to a predetermined value or less, when the cyclic diene-containing composition is used as a starting material for an aldehyde, it is possible to efficiently produce the aldehyde from the cyclic diene-containing composition while suppressing a decrease in catalytic activity of the hydroformylation reaction catalyst, and to recover expensive rhodium metal from the process liquid after the hydroformylation reaction at a high recovery rate.

The cyclic diene oxide in the present invention is a compound in which a functional group having oxygen is introduced into the above-described cyclic diene.

The "cyclic diene oxide" in the cyclic diene-containing composition and the method for producing the same of the present invention, the method for producing an aldehyde of the present invention, and the method for producing a cyclic diene-containing composition of the present invention can be treated as having the same meaning as the cyclic diene oxide in the present invention.

In the cyclic diene-containing composition of the present invention and the method for producing an aldehyde of the present invention, by setting the content proportion of the cyclic diene oxide contained in the cyclic diene-containing composition to a predetermined value or less, when the cyclic diene-containing composition is used as a starting material for an aldehyde such as an alicyclic aldehyde, it is possible to efficiently produce an aldehyde corresponding to the cyclic diene from the cyclic diene-containing composition, i.e., in a short reaction time and in a high yield, while suppressing a decrease in catalytic activity of the hydroformylation reaction catalyst, and to recover expensive rhodium metal from the process liquid after the hydroformylation reaction at a high recovery rate.

The cyclic diene oxide in the present invention is not particularly limited, and a compound having any skeleton selected from a tricyclodecene skeleton and a tricyclodecadiene skeleton and having an oxygen-containing functional group can be exemplified as a first embodiment.

The oxygen-containing functional group is not particularly limited, and at least one selected from the group consisting of a carbonyl group, a hydroxyl group, and an epoxy group can be exemplified.

More specifically, the first embodiment of the cyclic diene oxide in the present invention can include at least one selected from the group consisting of a compound having a carbonyl group and a tricyclodecadiene skeleton, a compound having a hydroxyl group and a tricyclodecadiene skeleton, and a compound having an epoxy group and a tricyclodecene skeleton.

The cyclic diene oxide in the present invention is not particularly limited, and at least one selected from the group consisting of a cyclic diene having a carbonyl group, a cyclic diene having a hydroxyl group, and a cyclic monoene having an epoxy group can be exemplified as a second embodiment.

The cyclic diene oxide in the present invention is not particularly limited, and an oxide of an unsaturated hydrocarbon compound can be exemplified as a third embodiment.

In the present invention, the "oxide of an unsaturated hydrocarbon compound" is a compound in which a functional group having oxygen is introduced into an unsaturated hydrocarbon compound.

Examples of the unsaturated hydrocarbon compound include the cyclic dienes in the present invention, isopropylnorbornene, and methyltetrahydroindene.

Specific examples of the oxide of the unsaturated hydrocarbon compound can include a compound having a carbonyl group, a compound having a hydroxyl group, and a compound having an epoxy group.

In the first embodiment, the second embodiment, and the third embodiment of the cyclic diene oxide in the present invention, in a case where the cyclic diene is dicyclopentadiene, more specifically, at least one selected from the group consisting of a compound in which one enantiomer is represented by General Formula (I) described below, a compound in which one enantiomer is represented by General Formula (II) described below, and a compound in which one enantiomer is represented by General Formula (III) described below can be exemplified.

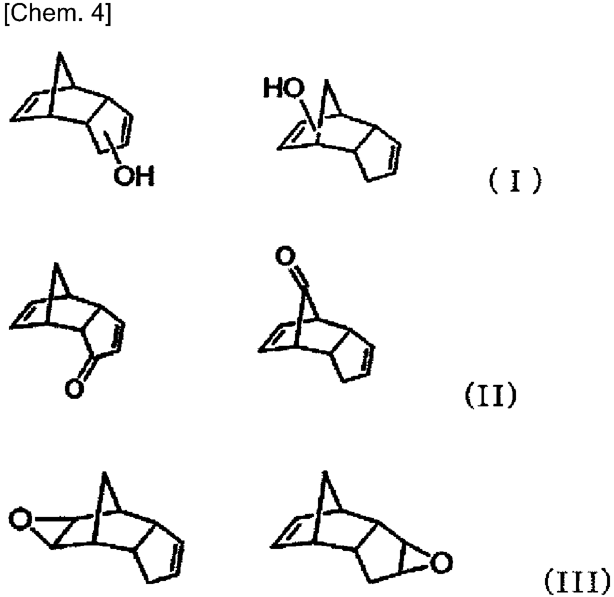

[In General Formula (I), a hydroxyl group (-OH) is bonded to any position in an illustrated dicyclopentadiene ring structure.]

For these cyclic diene oxides, one type may be used alone, or two or more types may be used in combination.

The content proportion of the cyclic diene oxide in the present invention can be measured by measurement method 1 described below.

### Measurement Method 1

The total content proportion of peaks for an elution time from 17.7 minutes to 18.8 minutes is measured by analysis under GC measurement conditions described below.

### GC Measurement Conditions

GC apparatus: gas chromatogram measuring apparatus (trade name: GC-2025, available from Shimadzu Corporation)
Detector: hydrogen flame ionization detector (FID)
Carrier gas: helium (column flow rate: 1.65 mL/min)
Column: capillary column (product name: DB-1, available from Agilent Technologies; size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C
Sample amount: 0.3 µL (split ratio: 1/30)

The upper limit of the content proportion of the cyclic diene oxide in the cyclic diene-containing composition of the present invention is 1.2 GC area% or less, preferably 0.5 GC area% or less, more preferably 0.35 GC area% or less, further preferably 0.25 GC area% or less, particularly preferably 0.14 GC area% or less, and most preferably 0.05 GC area% or less, from the viewpoint of efficiently producing an aldehyde corresponding to the cyclic diene from the cyclic diene-containing composition while suppressing a decrease in catalytic activity of the hydroformylation reaction catalyst using the cyclic diene-containing composition as a starting material, and recovering expensive rhodium metal from the process liquid after the hydroformylation reaction at a high recovery rate.

Meanwhile, the lower limit of the content proportion of the cyclic diene oxide is not particularly limited, and the cyclic diene-containing composition may be substantially free of the cyclic diene oxide (0.0 GC area%). However, from the viewpoint of economic efficiency such as production cost required for purification and separation of the cyclic diene-containing composition, the lower limit can be 0.0001 GC area% or more, and is preferably 0.0003 GC area% or more, more preferably 0.001 GC area% or more, further preferably 0.002 GC area% or more, particularly preferably 0.005 GC area% or more, and most preferably 0.01 GC area% or more, relative to the cyclic diene-containing composition.

The above upper and lower limits can be freely combined. For example, the content proportion of the cyclic diene oxide in the cyclic diene-containing composition is not particularly limited, and the cyclic diene-containing composition may be substantially free of the cyclic diene oxide (0.0 GC area%). Alternatively, the content proportion can be 0.0001 GC area% or more and 1.2 GC area% or less, and is preferably 0.0003 GC area% or more and 0.5 GC area% or less, more preferably 0.001 GC area% or more and 0.35 GC area% or less, further preferably 0.002 GC area% or more and 0.25 GC area% or less, particularly preferably 0.005 GC area% or more and 0.14 GC area% or less, and most preferably 0.01 GC area% or more and 0.05 GC area% or less.

### Peroxide

In the method for producing a cyclic diene-containing composition of the present invention and the method for producing an aldehyde of the present invention, in a case where the oxygen-containing compound is a peroxide, by setting the concentration of the peroxide contained in the cyclic diene-containing composition to a predetermined value or less, when the cyclic diene-containing composition is used as a starting material for an aldehyde such as an alicyclic aldehyde, an aldehyde corresponding to the cyclic diene can be efficiently produced by hydroformylation of the cyclic diene-containing composition while suppressing a decrease in catalytic activity of the hydroformylation reaction catalyst, even in a case where a procedure including recovery from the reaction liquid after the hydroformylation reaction and reuse is repeated.

Further, the cyclic diene-containing composition of the present invention preferably has a content proportion of the peroxide of 2.8 mmol/L or less.

In the method for producing a cyclic diene-containing composition of the present invention and the method for producing an aldehyde of the present invention, by setting the concentration of the peroxide contained in the cyclic diene-containing composition to a predetermined value or less, when the cyclic diene-containing composition is used as a starting material for an alicyclic aldehyde, an aldehyde corresponding to the cyclic diene can be efficiently produced by hydroformylation of the cyclic diene-containing composition while suppressing a decrease in catalytic activity of the hydroformylation reaction catalyst, even in a case where a procedure including recovery from the reaction liquid after the hydroformylation reaction and reuse is repeated.

The peroxide in the present invention is not particularly limited, and examples thereof can include a peroxide of an alkene compound.

Examples of the alkene compound include the cyclic diene in the present invention, isopropylnorbornene, and methyltetrahydroindene.

Specific examples of the peroxide of the alkene compound include a compound having a four-membered ring structure composed of two carbon molecules and two oxygen molecules, and a compound having a hydroperoxy group at the vinyl position.

More specific examples of the peroxide of the alkene compound include compounds represented by the following formulae in a case where the alkene compound is dicyclopentadiene, i.e., the cyclic diene.

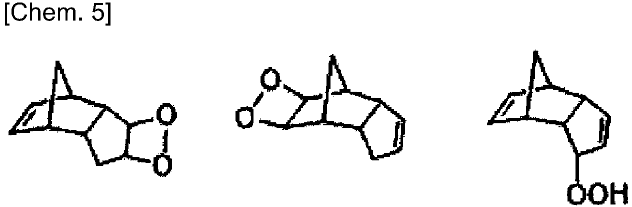

The concentration of the peroxide in the present invention can be measured by measurement method 1 described below. A more specific measurement method is as described in the Examples section below.

### Measurement Method 1

A mixed liquid of chloroform and acetic acid is added to a cyclic diene-containing composition under a nitrogen atmosphere, and a saturated aqueous potassium iodide solution is further added, followed by stirring and dilution with distilled water to prepare a measurement sample. The prepared measurement sample is subjected to iodometric titration using a 0.1 N aqueous sodium thiosulfate solution as a standard liquid, and the concentration (mmol/L) of the peroxide in the cyclic diene-containing composition is determined based on the amount of the standard liquid consumed.

The upper limit of the concentration of the peroxide in the cyclic diene-containing composition of the present invention is usually 2.8 mmol/L or less, preferably 2.0 mmol/L or less, more preferably 1.0 mmol/L or less, further preferably 0.6 mmol/L or less, and particularly preferably 0.3 mmol/L or less, from the viewpoint of efficiently producing an alicyclic aldehyde corresponding to the cyclic diene while suppressing a decrease in catalytic activity of the hydroformylation reaction catalyst, even in a case where a procedure including recovery from the reaction liquid after the hydroformylation reaction and reuse is repeated, when an aldehyde is produced by the hydroformylation reaction using the cyclic diene-containing composition as a starting material.

Meanwhile, the lower limit of the concentration of the peroxide is not particularly limited, and the cyclic diene-containing composition may be substantially free of the peroxide (0.0 mmol/L). However, the lower limit can be 0.001 mmol/L or more, and is preferably 0.005 mmol/L or more, more preferably 0.01 mmol/L or more, further preferably 0.02 mmol/L or more, and particularly preferably 0.03 mmol/L or more, relative to the cyclic diene-containing composition, from the viewpoint of economic efficiency such as production cost required for purification and separation of the cyclic diene-containing composition.

The above upper and lower limits can be freely combined. For example, the concentration of the peroxide in the cyclic diene-containing composition is not particularly limited, and the cyclic diene-containing composition may be substantially free of the peroxide (0.0 mmol/L). Alternatively, the concentration can be 0.001 mmol/L or more and 2.8 mmol/L or less, and is preferably 0.005 mmol/L or more and 2.0 mmol/L or less, more preferably 0.01 mmol/L or more and 1.0 mmol/L or less, further preferably 0.02 mmol/L or more and 0.6 mmol/L or less, particularly preferably 0.03 mmol/L or more and 0.3 mmol/L or less.

Examples of the methods for controlling the content proportion of the cyclic diene oxide in the cyclic diene-containing composition and further controlling the concentration of the peroxide include methods of controlling the amount of the cyclic diene oxide or the peroxide by an extraction method, a purification condition, or a distillation condition, a method of adding an antioxidant as described below, and a method of combining these methods.

### Method for Producing Cyclic Diene-Containing Composition

The method for producing a cyclic diene-containing composition in the present invention is not particularly limited, and for example, can include a method for producing the cyclic diene-containing composition of the present invention.

The method for producing the cyclic diene-containing composition of the present invention is a method for producing a cyclic diene-containing composition containing a cyclic diene by thermal decomposition of a hydrocarbon-containing composition to produce a hydrocarbon decomposition product; and distillation purification of the hydrocarbon decomposition product. The method is characterized by including setting the content proportion of the oxygen-containing compound of the present invention such as the cyclic diene oxide or the peroxide contained in the cyclic diene-containing composition to a predetermined threshold or less.

Examples of the method for setting the content proportion of the oxygen-containing compound of the present invention such as the cyclic diene oxide or the peroxide contained in the cyclic diene-containing composition to a predetermined threshold or less include a method for producing a cyclic diene-containing composition through thermal decomposition of a hydrocarbon-containing composition to produce a hydrocarbon decomposition product and distillation purification of the hydrocarbon decomposition product, as described below. In the distillation purification, it is preferable to control conditions for the distillation purification.

In this case, since the oxygen-containing compounds of the present invention such as the cyclic diene oxide and the peroxide described above are usually high-boiling-point components having a higher boiling point than the cyclic diene in the cyclic diene-containing composition of the present invention, these oxygen-containing compounds can be removed together with other high-boiling-point components, for example, in the distillation purification step in the method for producing a cyclic diene-containing composition of the present invention described below, and the content proportion thereof can be appropriately controlled.

Examples of the method for setting the content proportion of the oxygen-containing compound of the present invention such as the cyclic diene oxide or the peroxide contained in the cyclic diene-containing composition to a predetermined threshold or less include a method of adding an antioxidant to a hydrocarbon decomposition product obtained by thermal decomposition of a hydrocarbon-containing composition so that the content proportion of the oxygen-containing compound of the present invention such as the cyclic diene oxide or the peroxide contained in the cyclic diene-containing composition is a predetermined threshold or less.

That is, the hydrocarbon decomposition product obtained by thermal decomposition of the hydrocarbon-containing composition may contain an antioxidant.

In the method for producing a cyclic diene-containing composition of the present invention, when the hydrocarbon decomposition product contains an antioxidant, it is possible to suppress formation of an oxygen-containing compound such as a cyclic diene oxide or peroxide derived from a cyclic diene such as dicyclopentadiene due to oxidation of the cyclic diene.

The type and suitable amount of the antioxidant to be added will be described in detail in the section of the description of the method for producing an aldehyde of the present invention.

In the cyclic diene-containing composition of the present invention, a storage condition may be controlled so that the content proportion of the oxygen-containing compound of the present invention such as the cyclic diene oxide or the peroxide is the above-described threshold or less.

In this case, examples of the method for controlling the storage condition of the cyclic diene-containing composition include a method of adding an antioxidant to the cyclic diene-containing composition to be stored, and a method of controlling a dissolved oxygen concentration of the cyclic diene-containing composition to be stored. As in the case of adding an antioxidant, formation of a cyclic diene oxide or peroxide derived from a cyclic diene due to the oxidation of a cyclic diene such as dicyclopentadiene can be suppressed also by controlling the dissolved oxygen concentration.

In a case where an antioxidant is added to the cyclic diene-containing composition, the type of the antioxidant and the lower limit or upper limit of the amount of the antioxidant to be added have the same meanings as the type of an antioxidant and the lower limit or upper limit of the amount of the antioxidant to be added described in the method for producing an aldehyde of the present invention described below.

When the control of the storage condition includes controlling the dissolved oxygen concentration of the cyclic diene-containing composition to be stored, the method for controlling the dissolved oxygen concentration and the controlled concentration have the same meanings as the method for controlling the dissolved oxygen concentration and the dissolved oxygen concentration described in the method for producing an aldehyde of the present invention described below.

When the oxygen-containing compound is a cyclic diene oxide, the threshold of the content proportion of the cyclic diene oxide in the method for producing a cyclic diene-containing composition of the present invention is preferably 1.2 GC area% or less, more preferably 0.5 GC area% or less, further preferably 0.35 GC area% or less, particularly preferably 0.25 GC area% or less, especially preferably 0.14 GC area% or less, and most preferably 0.05 GC area% or less, as the content proportion of the cyclic diene oxide in the cyclic diene-containing composition to be produced.

When the oxygen-containing compound is a peroxide, the threshold of the peroxide concentration in the method for producing a cyclic diene-containing composition of the present invention is preferably 2.8 mmol/L or less, more preferably 2.0 mmol/L or less, further preferably 1.0 mmol/L or less, particularly preferably 0.6 mmol/L or less, and most preferably 0.3 mmol/L or less, as the concentration of the peroxide in the cyclic diene-containing composition to be produced.

The same applies to the threshold in the method for producing an aldehyde of the present invention described below.

In the method for producing a cyclic diene-containing composition of the present invention, production conditions and the like are controlled so as to produce a cyclic diene-containing composition having the above-described suitable oxygen-containing compound content proportion and cyclic diene content proportion.

### Method for Producing Cyclic Diene-Containing Composition

The method for producing a cyclic diene-containing composition of the present invention is not particularly limited, and examples thereof include a method in which a hydrocarbon decomposition product obtained by thermally decomposing a hydrocarbon-containing composition in treatment equipment for a hydrocarbon decomposition product such as ethylene production equipment described below is distilled and purified to produce the cyclic diene-containing composition, or a method in which the hydrocarbon decomposition product is separated and purified to produce a C5 hydrocarbon fraction described below, and the produced C5 hydrocarbon fraction is distilled and purified to produce the cyclic diene-containing composition.

By performing the distillation purification so that the content proportion of the oxygen-containing compound contained in the cyclic diene-containing composition is a predetermined threshold or less when the cyclic diene-containing composition is produced, it is possible to efficiently produce an alicyclic aldehyde corresponding to the cyclic diene from the cyclic diene-containing composition, i.e., in a short reaction time and in a high yield while suppressing a decrease in catalytic activity of the hydroformylation reaction catalyst, using the cyclic diene-containing composition as a starting material, and to recover expensive rhodium metal from the process liquid after the hydroformylation reaction at a high recovery rate.

As the hydrocarbon-containing composition, naphtha, coal, and natural gas can be used.

Among these, naphtha is preferred from the viewpoint of excellent productivity and quality of the resulting cyclic diene-containing composition.

### Ethylene Production Equipment

Ethylene production equipment in the present invention is production equipment for producing a C5 hydrocarbon fraction described below as one of separated and purified fractions of naphtha decomposition products. The ethylene production equipment refers to equipment configured to thermally decompose naphtha at a high temperature to form hydrogen, methane, ethane, ethylene, propane, propylene, hydrocarbons having 4 carbons such as butane or butadiene, hydrocarbons having 5 carbons, aromatic hydrocarbons such as benzene, other heavy oils, and the like, and then separate and purify them.

An embodiment of the ethylene production apparatus is not particularly limited and may be an apparatus having any structure as long as the apparatus includes distillation equipment for the C5 hydrocarbon fraction and performs separation and purification of the naphtha decomposition product. An example thereof is a configuration including equipment performing the following steps (1) to (4) sequentially.
Step (1): Thermal decomposition step of thermally decomposing a raw material, such as naphtha, coal, and natural gas in a decomposition furnace
Step (2): Rapid cooling step of rapidly cooling and separating the resultant decomposition gas
Step (3): Compression step of compressing the rapidly cooled and separated decomposition gas
Step (4): Purification step of separating and purifying the compressed decomposition gas into fractions, to thereby provide ethylene and propylene, which are main products

### C5 Hydrocarbon Fraction

The C5 hydrocarbon fraction in the present invention is a fraction containing a hydrocarbon having 5 carbons as a main component, and the fraction is obtained by thermal decomposition (cracking) of a hydrocarbon-containing composition, such as naphtha to provide a hydrocarbon decomposition product, separation of a heavy oil component from the hydrocarbon decomposition product, and then further separation and removal of hydrogen and hydrocarbons having from 1 to 4 carbons.

Specific examples of the C5 hydrocarbon fraction include mixtures containing C5 hydrocarbons such as isoprene, isopentane, normal pentane, and cyclopentadiene as a main component. The C5 hydrocarbon fraction contains a small amount of C4 hydrocarbon fraction and a small amount of C6 hydrocarbon fraction for separation performance. Furthermore, since the C5 hydrocarbon fraction contains cyclopentadiene, cyclopentadiene is polymerized and forms dicyclopentadiene as time passes, and thus the C5 hydrocarbon fraction contains dicyclopentadiene.

An example of the composition of the C5 hydrocarbon fraction is shown in Table 1. Typically, the C5 hydrocarbon fraction contains from 10 to 35 mass% of cyclopentadiene and dicyclopentadiene in total, although it also varies depending on the type of the hydrocarbon-containing composition such as naphtha which has been subjected to thermal decomposition.

**[Table 1]**

| Component | wt.% |
|---|---|
| Hydrocarbon having 4 or less carbons | 0 to 10 |
| Hydrocarbon having 5 carbons (e.g., isopentane, n-pentane, pentene-1, pentene-2, isoprene, pentadiene) | 65 to 90 |
| Cyclopentadiene | 10 to 35 |
| Dicyclopentadiene | |
| Hydrocarbon having 6 or more carbons | 0 to 30 |

### Distillation Purification of Hydrocarbon Decomposition Product or C5 Hydrocarbon Fraction

In the present invention, the distillation purification of the hydrocarbon decomposition product or the C5 hydrocarbon fraction may be performed by a single distillation operation, or may be performed by a combination of a plurality of distillation operations. In the case of performing a plurality of distillation operations in combination, a method of performing distillation operations under the same conditions a plurality of times, or a method of performing distillation operations under different conditions in combination two or more times can be employed.

In particular, from the viewpoint of setting the content proportion of the oxygen-containing compound of the present invention such as the cyclic diene oxide or the peroxide contained in the cyclic diene-containing composition of the present invention to a predetermined threshold or less, specifically, the content proportion of the cyclic diene oxide to 1.2 GC area% or less, or the concentration of the peroxide to 2.8 mmol/L or less, a method of performing distillation operations under the same conditions or different conditions in combination two or more times is preferable.

When the distillation operation is carried out a plurality of times, by using two or more distillation columns and controlling the number of theoretical plates or reflux ratio of each distillation column within the following range, separation and removal of the oxygen-containing compound, which is insufficient in a single distillation column, can be reliably achieved.

For example, in a case where two distillation columns are used in the distillation purification, i.e., in a case where a first distillation column and a second distillation column are included, it is preferable that the reflux ratio of the first distillation column is set to 20 or more and 30 or less, preferably 23 or more and 27 or less, and that the reflux ratio of the second distillation column is set to 1.8 or more and 4.5 or less, preferably 2.0 or more and 2.8 or less. In this manner, the oxygen-containing compound can be efficiently separated and removed, and the content proportion of the oxygen-containing compound can be efficiently controlled to the predetermined threshold or less.

Further, in the distillation purification, when the first distillation column and the second distillation column are included, it is preferable that the number of theoretical plates of the first distillation column is set to 22 or more and 28 or less, preferably 23 or more and 27 or less, and that the number of theoretical plates of the second distillation column is set to 10 or more and 20 or less, preferably 13 or more and 17 or less. By setting in this manner, the oxygen-containing compound can be efficiently separated and removed, and the content proportion of the oxygen-containing compound can be efficiently controlled to the predetermined threshold or less.

### Specific Embodiment of Method for Producing Cyclic Diene-Containing Composition

One specific embodiment of the method for producing a cyclic diene-containing composition of the present invention is a method for performing the following steps (I) to (IV) sequentially.

Step (I): A dimerization step of subjecting a hydrocarbon-containing composition such as naphtha to thermal decomposition to provide a C5 hydrocarbon fraction described below, introducing the C5 hydrocarbon fraction into a dimerization tank and heating the C5 hydrocarbon fraction to form a dimer of a cyclic diene precursor such as cyclopentadiene contained in the C5 hydrocarbon fraction, and producing a cyclic diene such as dicyclopentadiene corresponding to the cyclic diene precursor.

Step (II): A recovery step of transferring the resultant from step (I) to a recovery column and subjecting it to distillation to recover an unreacted component of step (I) from a column top, and taking out a C5 fraction heavy composition rich in the cyclic diene such as dicyclopentadiene from a column bottom.

Step (III): A low-boiling-point component removal step of transferring the C5 fraction heavy composition obtained from the column bottom in step (II) to a low-boiling-point component removal column and distilling it, removing a cyclic monoene such as vinylnorbornene produced as a byproduct at the time of the dimerization reaction of step (I) from a column top, wherein the removed cyclic monoene has a boiling point lower than that of the cyclic diene such as dicyclopentadiene, and taking out a fraction rich in the cyclic diene such as dicyclopentadiene from a column bottom.

Step (IV): A high-boiling-point component removal step of transferring the fraction rich in the cyclic diene such as dicyclopentadiene from a column bottom in step (III) to a high-boiling-point component removal column and distilling it, removing, from the column bottom, the oxygen-containing compound of the present invention, such as the above-described cyclic diene oxide or peroxide, having a boiling point higher than that of the cyclic diene such as dicyclopentadiene, and a high-boiling-point analogous codimer produced as a byproduct in the dimerization reaction in step (I), and taking out, from a column top, the cyclic diene-containing composition of the present invention containing the cyclic diene such as dicyclopentadiene in a high content proportion.

Steps (I) to (IV) will be described in more detail below.

### Step (I)

The C5 hydrocarbon fraction is firstly supplied to the dimerization step (I), and then dimerization of the cyclic diene precursor such as cyclopentadiene, contained in the C5 hydrocarbon fraction, is performed, to form a cyclic diene corresponding to the cyclic diene precursor, such as dicyclopentadiene.

The conditions for the dimerization are not particularly limited and are appropriately selected based on the content of the cyclic diene precursor such as cyclopentadiene, in the C5 hydrocarbon fraction, and the like; however, typically, the dimerization temperature is set to a range of from 50 to 110°C, the reaction time is set to a range of from 1 to 6 hours, and from 30 to 99 mass% of the cyclic diene precursor such as cyclopentadiene, contained in the C5 hydrocarbon fraction, is dimerized.

### Step (II)

The resultant product from the dimerization step (I) is then sent to the recovery step (II) of the unreacted component, and separation and recovery of the unreacted C5 hydrocarbon fraction are performed. This unreacted C5 hydrocarbon fraction contains isoprene, piperylene (i.e., 1,3-pentadiene), and the like, and these are respectively sent to separate purification processes as necessary.

For example, since the boiling point of the dicyclopentadiene that is the cyclic diene is 170°C and boiling points of other unreacted components are approximately from 30 to 50°C, the resultant product from the dimerization step (I) is transferred to a distillation column utilizing a boiling point difference between the dicyclopentadiene and the other unreacted components in this recovery step (II). The unreacted C5 hydrocarbon fraction is recovered from the column top of the distillation column, and the C5 fraction heavy composition rich in the cyclic diene such as dicyclopentadiene is taken out from the column bottom.

As the distillation condition, the distillation is typically performed at normal pressure; however, distillation under reduced pressure or steam distillation is performed to recover larger amounts of the C5 hydrocarbon fraction and the C6 hydrocarbon fraction.

### Step (III)

The C5 fraction heavy composition taken out from the column bottom of the distillation column in step (II) is, as necessary, liquefied by a condenser, and then sent to the low-boiling-point component removal step (III) (low-boiling-point component removal column) and subjected to distillation. The C5 fraction heavy composition sent to this step (III) is managed so as to set the content rate of the cyclic diene such as dicyclopentadiene, typically in a range of from 50 to 95 mass%.

From the column top of the low-boiling-point component removal column, a low-boiling-point component contained in the C5 fraction heavy composition is removed, and a fraction rich in the cyclic diene such as dicyclopentadiene is taken out from the column bottom.

Examples of the low-boiling-point component include, besides the C5 hydrocarbon fraction and the C6 hydrocarbon fraction that are not be recovered in the step (II), cyclic monoene such as vinylnorbornene, an analogous codimer such as propenyl norbornene obtained by codimerization of cyclopentadiene and piperylene, and the like. These low-boiling-point components have a lower boiling point than the dicyclopentadiene formed as byproducts at the time of the dimerization reaction in step (I).

Among these low-boiling-point components, cyclic monoene such as vinylnorbornene, the C5 hydrocarbon fraction, and the C6 hydrocarbon fraction are easily removed, but it is difficult to remove the analogous codimer component such as propenyl norbornene. Thus, as the low-boiling-point component removal column, a 50-to 100-staged distillation column is typically required.

The low-boiling-point component removal column is preferably controlled so as to set the content proportion of the cyclic diene such as cyclopentadiene to 0.5 GC area% or less and the content proportion of the hydrocarbon having 5 carbons to 2.0 GC area% or less in the column bottom outflow obtained from the column bottom.

The distillation condition in the low-boiling-point component removal column is controlled to the degree of pressure reduction in a range of from 5 to 200 torr, preferably from 10 to 50 torr, column bottom temperature in a range of from 50 to 120°C, preferably from 80 to 110°C, the number of theoretical plates in a range of 22 or more and 28 or less, preferably 23 or more and 27 or less, and the reflux ratio in a range of 20 or more and 30 or less, preferably 23 or more and 27 or less. Under these conditions, it is possible to efficiently remove the oxygen-containing compound of the present invention contained in the C5 fraction heavy composition obtained from the column bottom of the low-boiling-point component removal column in step (II) in the column bottom outflow obtained from the column bottom of the low-boiling-point component removal column, and to further remove 90 mass% or more of the cyclic monoene such as vinylnorbornene and the analogous codimer. Specifically, the content proportion of the cyclic diene such as cyclopentadiene can be set to 0.5 GC area% or less, and the content proportion of the hydrocarbon having 5 carbons can be adjusted to 2.0 GC area% or less in the column bottom distillate.

### Step (IV)

The fraction (column bottom liquid) rich in the cyclic diene such as dicyclopentadiene, taken out from the column bottom of the low-boiling-point component removal column in the low-boiling-point component removal step (III) is sent to the high-boiling-point component removal step (IV) (high-boiling-point component removal column) and subjected to distillation.

The high-boiling-point component removed in this step (IV) is the oxygen-containing compound of the present invention and a high-boiling-point analogous codimer such as methylbicyclononadiene obtained by dimerization of cyclopentadiene and isoprene, which have a higher boiling point than the cyclic diene such as dicyclopentadiene and are produced as byproducts at the time of the dimerization reaction in step (I). In a case where low polymers, such as a trimer of cyclopentadiene, are present, these low polymers are also removed.

The distillation condition in the high-boiling-point component removal column of step (IV), especially distillation temperature, affects the thermal decomposition of dicyclopentadiene and significantly affects the purity of the end product. Thus, attention is preferably paid to provide a targeted cyclic diene-containing composition.

The high-boiling-point component removal column is preferably a packed column to make pressure drop in the column as small as possible, and the high-boiling-point component removal column is controlled to have the decompression degree in a range of from 5 to 100 torr, preferably from 10 to 50 torr, the column bottom temperature in a range of from 50 to 120°C, preferably from 70 to 110°C, the number of theoretical plates in a range of 10 or more and 20 or less, preferably 13 or more and 17 or less, and the reflux ratio in a range of 1.8 or more and 4.5 or less, preferably 2.0 or more and 2.8 or less. Under these conditions, the cyclic diene-containing composition of the present invention, which contains a cyclic diene such as dicyclopentadiene in a high content proportion and in which the content proportion of the oxygen-containing compound of the present invention is appropriately controlled, is obtained from the column top of the high-boiling-point component removal column.

In the present invention, the high-boiling-point component removal column is preferably controlled so that, in the cyclic diene-containing composition obtained from the column top, the content proportion of the cyclic diene oxide as the oxygen-containing compound of the present invention is 1.2 GC area% or less, preferably the content proportion of cyclopentadiene or the like is 60 GC area% or more, and the content proportion of the cyclic diene oxide as the oxygen-containing compound of the present invention is 0.5 GC area% or less, more preferably 0.35 GC area% or less, further preferably 0.25 GC area% or less, particularly preferably 0.14 GC area% or less, and especially preferably 0.05 GC area% or less.

Alternatively, the high-boiling-point component removal column is preferably controlled so that, in the cyclic diene-containing composition obtained from the column top, the concentration of the peroxide as the oxygen-containing compound of the present invention is 2.8 mmol/L or less, preferably the concentration of cyclopentadiene or the like is 60 GC area% or more, and the concentration of the peroxide as the oxygen-containing compound of the present invention is 2.0 mmol/L or less.

The method for controlling the composition of the cyclic diene-containing composition obtained from the column top of the high-boiling-point component removal column to the above-described composition is, for example, a method for designing and manufacturing an apparatus so as to control the column bottom temperature of the high-boiling-point component removal column in a range of from 50 to 120°C, the degree of pressure reduction in a range of from 5 to 100 torr, the number of theoretical plates in a range of 10 or more and 20 or less, the reflux ratio in a range of 1.8 or more and 4.5 or less, and the retention time of a distillation column feed liquid in the column to 15 minutes or shorter, preferably 10 minutes or longer and 15 minutes or shorter.

When the column bottom temperature of the high-boiling-point component removal column is lower than 50°C, the content of the C5 hydrocarbon such as cyclopentadiene in the column bottom outflow liquid increases, whereas when the column bottom temperature exceeds 120°C, the content of a heavy fraction such as tricyclopentadiene in the column bottom outflow liquid increases, and in either case, the content of the cyclic diene such as dicyclopentadiene in the column bottom outflow liquid decreases.

By controlling the number of theoretical plates of the high-boiling-point component removal column to be in a range of 10 or more and 20 or less and the reflux ratio to be in a range of 1.8 or more and 4.5 or less, the oxygen-containing compound of the present invention can be efficiently separated and removed from the cyclic diene-containing composition of the present invention obtained from the column top of the high-boiling-point component removal column, and the content proportion of the oxygen-containing compound of the present invention can be controlled to the above-described predetermined threshold or less.

When the retention time of the distillation column feed liquid in the column exceeds 15 minutes, the oxygen-containing compound of the present invention may be formed in the column, or the heavy fraction such as tricyclopentadiene in the column bottom outflow liquid may increase.

When the retention time in the column is shorter than 10 minutes, the column bottom site of the distillation column becomes small, thereby making it difficult to install a meter for measuring the liquid level. Furthermore, the liquid amount held in the column bottom becomes smaller, the liquid inside the column bottom may be empty in a typical operation change of the distillation column, and thereby stable operation may be impaired.

Examples of the specific method for achieving the above-described retention time include designing the inner diameter of the column bottom of the distillation column in a size that suits the amount of the column bottom outflow component.

The control of the pressure of the distillation column described above may be performed by introduction of a gas free of a hydrocarbon having 5 or more carbons (pressure controlling gas) present in the treatment equipment of the naphtha decomposition product described above into the distillation column.

In the method for producing a cyclic diene-containing composition of the present invention, when the content proportion of the oxygen-containing compound of the present invention in the resulting cyclic diene-containing composition is controlled by adding an antioxidant to the hydrocarbon decomposition product obtained by the thermal decomposition of the hydrocarbon-containing composition, the antioxidant may be added in a necessary amount, for example, before performing step (II).

In the method for producing a cyclic diene-containing composition of the present invention, an antioxidant may be added to the produced cyclic diene-containing composition.

### One Embodiment of Production Equipment of Cyclic Diene-Containing Composition

Next, one embodiment of production equipment of the cyclic diene-containing composition of the present invention will be described with reference to a drawing illustrating this embodiment.

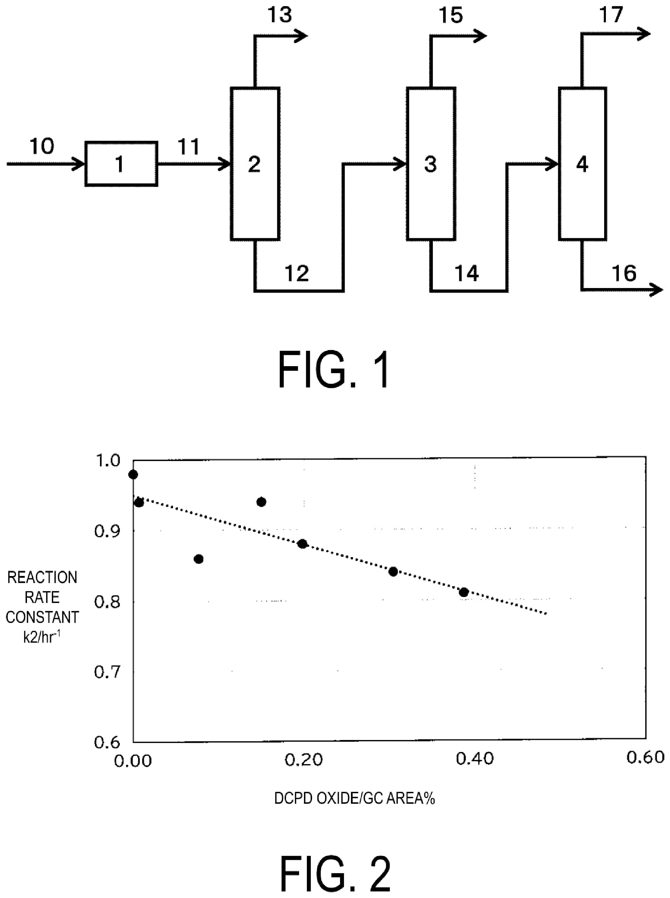

In FIG. 1, the C5 hydrocarbon fraction obtained by thermal decomposition of the hydrocarbon-containing composition such as naphtha, is firstly supplied to a dimerization tank 1 through a tube 10. In this dimerization tank 1, dimerization of a cyclic diene precursor such as cyclopentadiene is performed, and thus a cyclic diene such as dicyclopentadiene corresponding to the cyclic diene precursor is formed [dimerization step (I)]. After the dimerization reaction, the content of the dimerization tank 1 is sent to a distillation column 2 through a tube 11 [recovery step (II) of an unreacted component] and subjected to distillation.

In the distillation column 2, the unreacted C5 hydrocarbon fraction is removed from the column top of the distillation column 2 through a tube 13, a column bottom liquid rich in the cyclic diene such as dicyclopentadiene (C5 fraction heavy composition) is sent from the column bottom of the distillation column 2 through a tube 12 to a low-boiling-point component removal column 3 [low-boiling-point component removal step (III)] and subjected to distillation.

In the low-boiling-point component removal column 3, the cyclic monoene such as vinylnorbornene, the C5 hydrocarbon fraction and the C6 hydrocarbon fraction, and the analogous codimer, all of which respectively have a lower boiling point than the cyclic diene such as cyclopentadiene, are removed from the column top of the low-boiling-point component removal column 3 through a tube 15, a fraction rich in the cyclic diene such as dicyclopentadiene is sent from the column bottom of the low-boiling-point component removal column 3 through a tube 14 to a high-boiling-point component removal column 4, which is the last [high-boiling-point component removal step (IV)] and subjected to distillation.

In the high-boiling-point component removal column 4, the high-boiling-point oxygen-containing compound of the present invention and the high-boiling-point analogous codimer, which have a higher boiling point than the cyclic diene such as dicyclopentadiene, and the remaining low polymer are removed from the column bottom of the high-boiling-point component removal column 4 through a tube 16, and the cyclic diene-containing composition containing the cyclic diene such as dicyclopentadiene in a high content proportion is obtained from the column top of the high-boiling-point component removal column 4 through a tube 17.

The order of performing the aforementioned step (I) to step (IV), especially step (II) to step (IV), is not particularly limited, and the order of performing these steps can be changed as long as the effects of the present invention are not impaired.

### Method for Producing Aldehyde

The method for producing an aldehyde of the present invention is a method for producing an aldehyde by subjecting a cyclic diene in the cyclic diene-containing composition to a hydroformylation reaction.

The method for producing an aldehyde of the present invention includes setting the content proportion of the oxygen-containing compound of the present invention such as the cyclic diene oxide or the peroxide contained in the cyclic diene-containing composition to a predetermined threshold or less.

The cyclic diene in the method for producing an aldehyde of the present invention can be treated as having the same meaning as the cyclic diene in the cyclic diene-containing composition of the present invention described above.

In the method for producing an aldehyde of the present invention, the cyclic diene-containing composition of the present invention can be used as the cyclic diene-containing composition described above.

The method for setting the content proportion of the oxygen-containing compound of the present invention such as the cyclic diene oxide or the peroxide contained in the cyclic diene-containing composition to a predetermined threshold or less is not particularly limited, and examples thereof include a method in which a hydrocarbon decomposition product obtained by thermally decomposing a hydrocarbon-containing composition such as naphtha is distilled and purified to produce the cyclic diene-containing composition as described above. In the distillation purification, it is preferable to control conditions for the distillation purification.

In the method for producing an aldehyde of the present invention, the hydrocarbon decomposition product may contain an antioxidant as described above in the method for producing a cyclic diene-containing composition of the present invention.

The antioxidant in the method for producing an aldehyde of the present invention can be treated as having the same meaning as the antioxidant in the method for producing a cyclic diene-containing composition of the present invention.

When the hydrocarbon decomposition product contains an antioxidant, it is possible to suppress the formation of the oxygen-containing compound of the present invention such as the above-described cyclic diene oxide or peroxide derived from a cyclic diene such as dicyclopentadiene due to the oxidation of the cyclic diene.

Furthermore, in the method for producing an aldehyde of the present invention, as described above in the method for producing a cyclic diene-containing composition of the present invention, an antioxidant can be added to the hydrocarbon decomposition product so that the content proportion of the oxygen-containing compound of the present invention such as the cyclic diene oxide or the peroxide contained in the cyclic diene-containing composition is a predetermined threshold or less. When an antioxidant is added to the hydrocarbon decomposition product, it is possible to suppress the formation of the oxygen-containing compound of the present invention such as the above-described cyclic diene oxide or peroxide derived from a cyclic diene such as dicyclopentadiene due to the oxidation of the cyclic diene.

In the method for producing an aldehyde of the present invention, a storage condition may be controlled so that the content proportion of the oxygen-containing compound of the present invention such as the cyclic diene oxide or the peroxide contained in the cyclic diene-containing composition is the threshold or less.

In this case, examples of the method for controlling the storage condition of the cyclic diene-containing composition include a method of adding an antioxidant to the cyclic diene-containing composition to be stored, and a method of controlling a dissolved oxygen concentration of the cyclic diene-containing composition to be stored. As in the case of adding an antioxidant, the formation of the oxygen-containing compound of the present invention such as the above-described cyclic diene oxide or peroxide derived from a cyclic diene such as dicyclopentadiene due to the oxidation of the cyclic diene can also be suppressed by controlling the dissolved oxygen concentration.

The type of the antioxidant in the present invention is not particularly limited as long as the antioxidant has excellent dispersibility and solubility in the reaction system and can suppress the oxidation of the cyclic diene and the formation of the oxygen-containing compound of the present invention such as the cyclic diene oxide or the peroxide derived from the cyclic diene. For example, a person skilled in the art can appropriately select and use a known phenol-based compound, a known sulfur-based compound, a known phosphorus-based compound, a known amine-based compound, or the like used as an antioxidant. Among these, a phenol-based compound, particularly a hindered phenol-based compound is preferable, and it is preferable to use at least one of these.

Examples of the phenol-based compound include t-butylcatechol, 2,6-di-tert-butyl-4-hydroxytoluene (BHT), N,N'-(1,6-hexanediyl)bis[3,5-bis(1,1-dimethylethyl)-4-hydroxybenzenepropanamide], pentaerythritol tetrakis[3-[3,5-di(t-butyl)-4-hydroxyphenyl]propionate], 2,2 thio-[diethylbis-3(3,5-di-t-butyl-4-hydroxyphenyl)propionate], octadecyl 3-(3,5-di-t-butyl-4-hydroxyphenyl)propionate, 4,4',4"-[(2,4,6-trimethylbenzene-1,3,5-triyl)tris(methylene)]tris(2,6-di-t-butylphenol), bis(3-t-butyl-4-hydroxy-5-methylbenzenepropanoic acid)ethylenebis(oxyethylene), 1,3,5-tris(3,5-di-t-butyl-4-hydroxybenzyl)isocyanuric acid, and 3,9-bis[2-[3-(3-t-butyl-4-hydroxy-S-methylphenyl)propionyloxy]-1,1-dimethylethyl]-2,4,8,10-tetraoxaspiro(5,5)undecane.

One type of these antioxidants may be used alone, or two or more types thereof may be used in combination.

When the hydrocarbon decomposition product contains an antioxidant, or when an antioxidant is added to the hydrocarbon decomposition product, the lower limit of the content or the amount of the antioxidant added is not particularly limited as long as the content proportion of the oxygen-containing compound of the present invention such as the cyclic diene oxide or the peroxide can be set to the above-described threshold or less. As an example, the content of the antioxidant in the hydrocarbon decomposition product is preferably 30 ppm by mass or more, more preferably 50 ppm by mass or more, and further preferably 100 ppm by mass or more.

Meanwhile, when an antioxidant is added to the hydrocarbon decomposition product, the upper limit of the content or the amount of the antioxidant added is not particularly limited as long as the quality of the cyclic diene-containing composition of the present invention can be maintained well and an increase in production cost spent for purification and the like can be kept within an acceptable range. As an example, the content of the antioxidant in the hydrocarbon decomposition product is preferably 1000 ppm by mass or less, more preferably 500 ppm by mass or less, and further preferably 300 ppm by mass or less.

The above upper and lower limits can be freely combined.

When the cyclic diene-containing composition according to the present invention contains an antioxidant, or when an antioxidant is added to the cyclic diene-containing composition, the lower limit of the content or the amount of the antioxidant added is not particularly limited as long as the content proportion of the oxygen-containing compound of the present invention such as the cyclic diene oxide or the peroxide can be set to the above-described threshold or less. As an example, the content of the antioxidant in the cyclic diene-containing composition is preferably 30 ppm by mass or more, more preferably 50 ppm by mass or more, and further preferably 100 ppm by mass or more.

Meanwhile, when the cyclic diene-containing composition contains an antioxidant, the upper limit of the content or the amount of the antioxidant added is not particularly limited as long as the quality of the cyclic diene-containing composition of the present invention can be maintained well and an increase in production cost spent for purification and the like can be kept within an acceptable range. As an example, the content of the antioxidant in the cyclic diene-containing composition is preferably 1000 ppm by mass or less, more preferably 500 ppm by mass or less, and further preferably 300 ppm by mass or less.

The above upper and lower limits can be freely combined.

When the content of the antioxidant in the hydrocarbon decomposition product or the cyclic diene-containing composition is equal to or more than the lower limit described above, the formation of the oxygen-containing compound can be effectively inhibited by a sufficient amount of the antioxidant, and the content proportion of the oxygen-containing compound of the present invention such as the cyclic diene oxide or the peroxide described above can be readily reduced to the threshold or less.

Meanwhile, when the content of the antioxidant is equal to or less than the upper limit described above, the quality of the cyclic diene-containing composition of the present invention can be maintained well, and an increase in production costs spent for purification and the like can be kept within an acceptable range. Further, the risk of the antioxidant precipitating at the column bottom of the distillation column and clogging pipes can be reduced.

In the method for producing an aldehyde of the present invention, when the dissolved oxygen concentration of the cyclic diene-containing composition to be stored is controlled so that the content proportion of the oxygen-containing compound of the present invention such as the cyclic diene oxide or the peroxide contained in the cyclic diene-containing composition is the threshold or less, the dissolved oxygen concentration can be controlled by, for example, circulating an inert gas such as nitrogen in the cyclic diene-containing composition or under a storage atmosphere for the cyclic diene-containing composition.

Also in this case, the controlled concentration of oxygen dissolved in the cyclic diene-containing composition is not particularly limited, and may be any concentration as long as the content proportion of the oxygen-containing compound of the present invention such as the cyclic diene oxide or the peroxide in the cyclic diene-containing composition can be set to the above-described threshold or less, and the dissolved oxygen concentration of the cyclic diene-containing composition is preferably 100 ppm by volume or less, and particularly preferably 50 ppm by volume or less, from the viewpoint of the effect of suppressing the formation of the oxygen-containing compound. Meanwhile, with respect to excessively reducing the dissolved oxygen concentration, the dissolved oxygen concentration in the cyclic diene-containing composition is usually 1 ppm by volume or more from the viewpoint of economic efficiency such as production cost required for reducing the dissolved oxygen concentration.

In the method for producing an aldehyde of the present invention, the method for producing an aldehyde through the hydroformylation reaction is not particularly limited and can be performed in accordance with an ordinary method.

For example, in accordance with the method described in JP 2001-10999 A, in a hydroformylation reaction solvent consisting of a hydrocarbon compound, in the coexistence with a catalyst composed of a rhodium compound and an organophosphorus compound, the cyclic diene contained in the cyclic diene-containing composition of the present invention is subjected to hydroformylation by using hydrogen and carbon monoxide, and thus an aldehyde can be produced.

Specifically, when the cyclic diene in the cyclic diene-containing composition of the present invention is dicyclopentadiene, in accordance with the method described in JP 2001-10999 A, in the hydroformylation reaction solvent consisting of the hydrocarbon compound, in the coexistence with the catalyst consisting of the rhodium compound and the organophosphorus compound, the dicyclopentadiene contained in the cyclic diene-containing composition as described in the following reaction formula (I) is subjected to hydroformylation by using hydrogen and carbon monoxide, and thus tricyclodecane dicarbaldehyde can be produced.

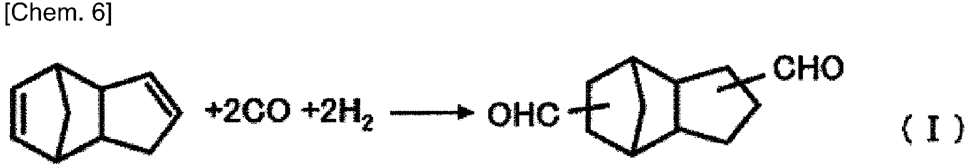

The rhodium compound used in this hydroformylation step forms a complex with the organophosphorus compound and does not depend on a precursor of the rhodium compound as long as it exhibits hydroformylation activity in the presence of hydrogen and carbon monoxide.

As the rhodium compound, a rhodium metal hydride carbonyl phosphorus complex having catalytic activity may be formed in a reaction vessel by introducing a catalyst precursor substance, such as Rh(acac)(CO)₂, Rh₂O₃, Rh₄(CO)₁₂, Rh₆(CO)₁₆, or Rh(NO₃)₃, together with the organophosphorus compound into a reaction mixture. Alternatively, a previously prepared rhodium metal hydride carbonyl phosphorus complex catalyst may be introduced into a reaction vessel.

In a specific preferred example of the present invention, Rh(acac)(CO)₂ is used as a rhodium precursor substance and reacted with the organophosphorus compound in the presence of a solvent and then introduced into a reactor together with an excessive amount of a free organophosphorus compound, and thus a rhodium-organophosphorus complex catalyst having catalytic activity is formed.

Examples of the organophosphorus compound forming the catalyst of a hydroformylation reaction with the rhodium compound include known phosphites and known phosphines.

Among the phosphites, a compound represented by general formula P (-OR¹¹)(-OR¹²)(-OR¹³) (in the formula, R¹¹, R¹², and R¹³ each represent an optionally substituted aryl group or alkyl group) is preferable. Specific examples of R¹¹, R¹², and R¹³ include an aryl group, such as a phenyl group or a naphthyl group, optionally substituted with a methyl group, an ethyl group, an isopropyl group, an n-butyl group, a t-butyl group, a methoxy group, or the like; an aliphatic alkyl group such as a methyl group, an ethyl group, an isopropyl group, an n-butyl group, or a t-butyl group; and an alicyclic alkyl group such as a cyclopentyl group or a cyclohexyl group optionally substituted with a lower alkyl group such as a methyl group, an ethyl group, an isopropyl group, an n-butyl group, or a t-butyl group.

Specific examples of the preferred phosphite include tris(2-t-butylphenyl) phosphite, tris(3-methyl-6-t-butylphenyl) phosphite, tris(3-methoxy-6-t-butylphenyl) phosphite, tris(2,4-di-t-butylphenyl) phosphite, and di(2-t-butylphenyl)(t-butyl) phosphite. The phosphite is not limited to the above examples. One of these phosphites may be used alone, or two or more of these phosphites may be used in combination.

As the phosphine, a sterically-hindered alkylphosphine is effective for the hydroformylation reaction of the dicyclopentadiene. Specific examples thereof include tricyclopropylphosphine, tricyclobutylphosphine, tricyclopentylphosphine, tricyclohexylphosphine, tricycloheptylphosphine, and tricyclooctylphosphine.

The phosphine is not limited to the above examples. One of these phosphines may be used alone, or two or more of these phosphines may be used in combination.

The amount of the organophosphorus compound to be used is not particularly limited and can be appropriately set according to known technologies by a person skilled in the art.

The amount of the rhodium catalyst to be used is also not particularly limited and can be appropriately set according to known technologies by a person skilled in the art.

The hydroformylation reaction of the dicyclopentadiene can be performed without using any solvent; however, the hydroformylation reaction can be more suitably performed when an organic solvent inactive in the reaction is used.

The temperature and the pressure of the hydroformylation reaction of the dicyclopentadiene are not particularly limited and can be appropriately set according to known technologies by a person skilled in the art. The reaction temperature is typically from 40 to 160°C, and preferably from 80 to 140°C, and the reaction pressure is typically from 1 to 15 MPa.

The molar ratio of hydrogen and carbon monoxide in a gas mixture of hydrogen/carbon monoxide used in the reaction is not particularly limited and can be appropriately set according to known technologies by a person skilled in the art. Typically, as an introduction gas composition, the molar ratio of the hydrogen to the carbon monoxide (hydrogen/carbon monoxide) can be set to fall within a range of 0.2 to 5.0.

As the reaction method of the hydroformylation, a continuous feeding method is employed. In the continuous feeding method, the hydroformylation is performed, while the cyclic diene-containing composition of the present invention itself as a raw material or a mixed solution of the cyclic diene-containing composition of the present invention and a solvent is supplied to a reactor including the rhodium-organophosphorus complex catalyst, a solvent, and the gas mixture of hydrogen and carbon monoxide. The use of this method can suppress thermal decomposition of the dicyclopentadiene that is the cyclic diene in the cyclic diene-containing composition of the present invention in the reactor, and formation of cyclopentadiene that inhibits the hydroformylation reaction, and thus reaction rate and yield can be maintained well. To maintain the fluidity of the cyclic diene-containing composition of the present invention, it is preferably diluted with the solvent described above, and the diluted cyclic diene-containing composition is preferably supplied to the reactor at a temperature that does not allow depolymerization of the composition and that does not allow generation of cyclopentadiene.

### Extraction of Tricyclodecane Dicarbaldehyde

The method of extraction and separation of tricyclodecane dicarbaldehyde as a product from the reaction product liquid after completion of the hydroformylation reaction is not particularly limited and can be appropriately set according to known technologies by a person skilled in the art.

For example, the reaction product liquid obtained after the hydroformylation reaction or a diluted product liquid obtained by diluting the reaction product liquid using a hydrocarbon compound usable as a hydroformylation reaction solvent is mixed with an extraction solvent, and then the mixture is separated into layers, whereby tricyclodecane dicarbaldehyde as a product in the reaction product liquid can be extracted into an extraction solvent layer.

The extraction solvent is not particularly limited, and examples thereof include alcohols.

The alcohol is not particularly limited, and examples thereof include primary alcohols having from 1 to 3 carbons and polyhydric alcohols having from 2 to 6 carbons. Examples of the primary alcohol include methanol, ethanol, and propanol. Examples of the polyhydric alcohol having from 2 to 6 carbons include ethylene glycol, 1,3-propanediol, 1,2-propanediol, 1,4-butanediol, 1,2-butanediol, 1,3-butanediol, 2,3-butanediol, isomers of pentanediol, neopentyl glycol, hexanediol, glycerin, pentaerythritol, and trimethylolpropane.

Among these, methanol, ethylene glycol, propanediol, and butanediol are suitably used because they have a relatively low boiling point, are available at a relatively low cost, are liquid, and have excellent handleability.

One of these alcohols may be used alone or two or more of these alcohols may be used in combination.

The extraction may be performed in the presence of water in addition to the alcohol. The addition of water facilitates distribution of the aldehyde and the catalyst component into each layer.

The hydroformylation reaction solvent and the extraction solvent are preferably excellent in liquid separation property from the viewpoint of efficiently extracting the product tricyclodecane dicarbaldehyde from the hydroformylation reaction solvent, and specifically, there is preferably a significant difference in density between both solvents. From this viewpoint, examples of a combination of the hydroformylation reaction solvent and the extraction solvent include methylcyclohexane and ethylene glycol, and methylcyclohexane and a mixed solution of water and methanol.

The volume ratio of the extraction solvent to the reaction product liquid is not particularly limited, and can be determined based on, for example, the solubility of tricyclodecane dicarbaldehyde in the extraction solvent and the content of tricyclodecane dicarbaldehyde in the reaction product liquid.

For example, when tricyclodecane dicarbaldehyde to be separated has a high solubility in the extraction solvent and is present at a low concentration in the reaction product liquid, tricyclodecane dicarbaldehyde can be effectively extracted by using the extraction solvent at a low volume ratio (extraction solvent/reaction product liquid). As the concentration of the product increases, the volume ratio (extraction solvent/reaction product liquid) for extracting tricyclodecane dicarbaldehyde from the reaction product liquid increases.

Meanwhile, when tricyclodecane dicarbaldehyde has a relatively low solubility in the extraction solution, the volume ratio can be appropriately selected from a range of 10:1 to 1:10. In order to increase the amount of tricyclodecane dicarbaldehyde to be extracted using a small amount of extraction solvent, it is effective to divide the extraction solvent and perform the extraction operation several times. In an extraction operation in the final stage, a hydroformylation reaction solvent such as methylcyclohexane may be added to the reaction product liquid in an amount of about from 5 to 20 mass%, whereby the removal rate of the catalyst can be improved by the addition of the hydroformylation reaction solvent.

The temperature of the extraction operation is not particularly limited, and can be set to a hydroformylation reaction temperature or lower.

The method of the extraction operation is not particularly limited, and examples thereof include a method of adding the extraction solvent to a hydroformylation reactor after the hydroformylation reaction to perform extraction treatment, and a method of putting the hydroformylation reaction product liquid extracted from the hydroformylation reactor into an extraction tank to perform extraction treatment.

A layer containing the reaction product liquid, which is recovered after the extraction operation, contains the hydroformylation reaction catalyst and the hydroformylation reaction solvent. Therefore, this layer can be returned to the hydroformylation reactor and reused. In this case, rhodium, which is a catalyst component, an organophosphorus ligand, or a solvent may be added to the layer to be reused. A process of the extraction operation described above can be carried out as both a batch process and a continuous process.

In the cyclic diene-containing composition of the present invention used as a raw material for producing an aldehyde in the present invention, the content proportion of the oxygen-containing compound of the present invention such as the cyclic diene oxide or the peroxide is a predetermined threshold or less, and thus, it is possible to recover expensive rhodium metal from the process liquid after the hydroformylation reaction at a high recovery rate, and to repeatedly perform an efficient hydroformylation reaction by reusing the recovered rhodium metal as a catalyst.

### Method for Producing Alcohol

The method for producing an alcohol of the present invention is a method in which an aldehyde is produced by the method for producing an aldehyde of the present invention, and an alcohol is produced from the aldehyde.

The method for producing an alcohol from the aldehyde is not particularly limited, and can be performed in accordance with an ordinary method. For example, according to the method described in JP 2001-10999 A, an alcohol can be produced by subjecting the aldehyde produced by the method for producing an aldehyde of the present invention to a known hydrogenation reaction as it is, or by dimerizing the produced aldehyde and then subjecting the dimerized product to a known hydrogenation reaction.

Specifically, when the cyclic diene in the cyclic diene-containing composition of the present invention is dicyclopentadiene, the dicyclopentadiene contained in the cyclic diene-containing composition is subjected to hydroformylation to produce tricyclodecane dicarbaldehyde, and then the produced tricyclodecane dicarbaldehyde is subjected to a hydrogenation reaction in the presence of a hydrogenation catalyst and hydrogen, whereby tricyclo[5.2.1.0^{2,6}]decanedimethanol can be produced.

As the hydrogenation catalyst used in the hydrogenation reaction, a known solid catalyst in which a metal such as Ru, Ni, Cr, or Cu is supported on a carrier can be used.

The hydrogenation reaction is usually carried out at a temperature from 60 to 200°C and a hydrogen pressure of about from 0.1 to 20 MPaG.

### Examples

Hereinafter, the present invention will be more specifically described with reference to experimental examples in place of examples below, but the present invention is not limited to the experimental examples below as long as the gist of the present invention is not deviated.

The following experimental examples and the like are merely illustrative and are not intended to limit any of the embodiments described herein. The following experimental examples do not limit the present invention in any way.

Values of various production conditions and evaluation results in the following experimental examples and the like have significance as preferable values of upper limits or lower limits in the embodiments of the present invention, and a preferable range may be a range defined by a combination of the value of an upper limit or lower limit described above and a value in the following experimental examples or values between the experimental examples.

The compounds used in the following experimental examples and the like are as follows.
DCPD: dicyclopentadiene (available from Mitsubishi Chemical Corporation)
Rhodium acetylacetonate dicarbonyl (trade name: Rh(acac)(CO)₂,
available from N.E. Chemcat Corporation)
Nickel-supported diatomaceous earth catalyst (nickel-chromium-supported diatomaceous earth having a nickel support amount of 12 mass% and a chromium support amount of 2 mass%, produced in accordance with the description of Experimental Example 1 of JP 2005-279587 A)
DBPO: (trade name: tris(2,4-di-tert-butylphenyl) phosphite, available from Tokyo Chemical Industry Co., Ltd.)
Methylcyclohexane (trade name: methylcyclohexane, available from FUJIFILM Wako Pure Chemical Corporation)
Methanol (trade name: methanol, available from FUJIFILM Wako Pure Chemical Corporation)

### Reference Experimental Example 1

### Production of Heavy Oil by Thermal Decomposition of Naphtha

Using a commercially available naphtha as an hydrocarbon-containing composition and a naphtha decomposition plant available from Mitsubishi Chemical Corporation, a hydrocarbon decomposition product was produced by subjecting the naphtha to thermal decomposition by the following procedure, in accordance with a method for thermal decomposition of naphtha disclosed in JP 7-39354 B.

The produced hydrocarbon decomposition product was supplied to a distillation column and subjected to fractional distillation, and a C5 hydrocarbon fraction containing a hydrocarbon having 5 carbons as a main component was recovered.

Subsequently, the resultant C5 hydrocarbon fraction was supplied to a reaction distillation column. While cyclopentadiene contained in the C5 hydrocarbon fraction was dimerized and converted into dicyclopentadiene, separation and purification were performed under the following conditions. Thus, a C5 fraction heavy composition containing dicyclopentadiene, i.e., a cyclic diene was recovered from the column bottom of the distillation column.

### Distillation Purification of Heavy Oil

In a distillation column having 40 plates, 1895 g of the resultant C5 hydrocarbon fraction was charged, a fractional distillation operation was performed under the following fractional distillation conditions, and the distillate from the column top of the distillation column was fractionated into about 100-g fractions.

### Fractional Distillation Conditions

Column bottom temperature: 40 to 140°C
Column top temperature: 24 to 80°C
Pressure: 1 to 10 kPa (7.5 to 75 torr)
Retention time of the distillation column feed liquid in the column: approximately 19 hours

The fractionated fractions were analyzed under the following GC measurement conditions using a gas chromatograph (GC) measuring apparatus and a method using total area in gas chromatography, and a fraction in which the content proportion of DCPD was 99% (hereinafter, referred to as "DCPD having a purity of 99%") was selected.

### GC Measurement Conditions

GC apparatus: GC-2025 (high-performance general-purpose gas chromatograph, available from Shimadzu Corporation)
Detector: hydrogen flame ionization detector (FID)
Carrier gas: helium (column flow rate: 1.65 mL/min)
Column: Capillary column DB-1 (available from Agilent Technologies; size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C
Sample amount: 0.3 µL (split ratio: 1/30)

The compositional proportion of each component in the method using total area in gas chromatography was calculated as an area content (unit: GC area%) of each peak component when the total area of GC peaks of all products observed in a temperature condition region of the retention time of from 2.5 to 30 minutes in the gas chromatogram was taken as 100%. Further, the GC area ratio of the total area content (unit: GC area%) of DCPD oxides described below to the area content (unit: GC area%) of DCPD in each peak component was calculated.

### Reference Experimental Example 2

### Distillation Purification of Dicyclopentadiene (DCPD)

As a cyclic diene, 1494.1 g of the above-described DCPD having a purity of 99% was charged in a single distillation column made of glass, and distillation was performed under conditions of a pressure of from 1 to 0.1 kPa, a column bottom temperature of from 70 to 90°C, and a column top temperature of from 60 to 70°C, and the distillate from the column top of the distillation column was fractionated into 200-g fractions. Thus, distillates 1 to 7 shown in Table 2 were obtained in the order of distillation, and used as DCPD-containing compositions.

The total content proportion of the DCPD oxides as cyclic diene oxides contained in each of the DCPD-containing compositions, and the content proportions of a compound in which one enantiomer constituting the DCPD oxides is represented by General Formula (I) above (referred to as "compound (I)"), a compound in which one enantiomer constituting the DCPD oxides is represented by General Formula (II) above (referred to as "compound (II)"), and a compound in which one enantiomer constituting the DCPD oxides is represented by General Formula (III) above (referred to as "compound (III)") were measured according to measurement method 1 described below.

### Measurement Method 1

The total content proportion of peaks for an elution time from 17.7 minutes to 18.8 minutes was measured by analysis under GC measurement conditions described below.

### GC Measurement Conditions

GC apparatus: gas chromatogram measuring apparatus (trade name: GC-2025, available from Shimadzu Corporation)
Detector: hydrogen flame ionization detector (FID)
Carrier gas: helium (column flow rate: 1.65 mL/min)
Column: capillary column (product name: DB-1, available from Agilent Technologies; size: length 30 m × inner diameter 0.25 mm, film thickness 1.00 µm)
Column temperature: 50°C (retention time: 5 minutes) → temperature increase at 10°C/min → 300°C (retention time: none)
Inlet temperature: 200°C
Detector temperature: 300°C
Sample amount: 0.3 µL (split ratio: 1/30)

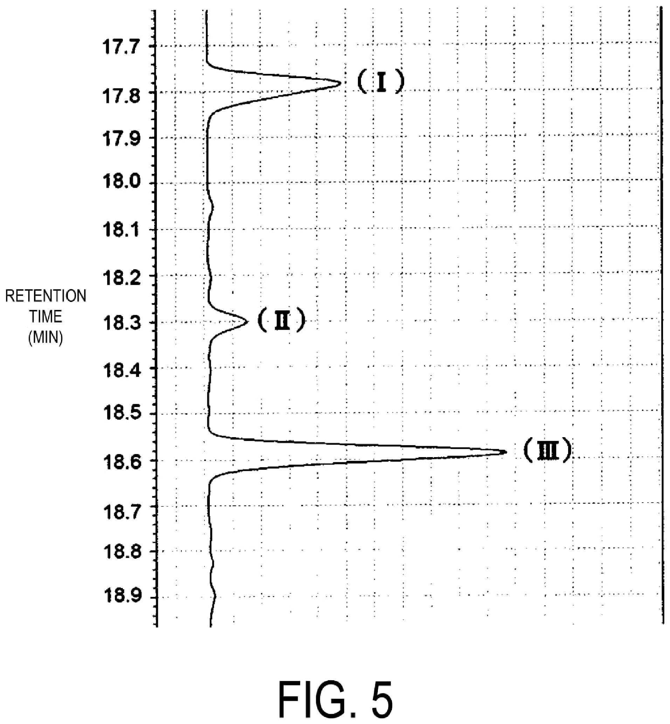

FIG. 5 illustrates a gas chromatogram of a DCPD-containing composition produced in Reference Experimental Example 2.

A peak (1) for an elution time from 17.73 to 17.88 minutes is a peak of the compound (I), a peak (2) for an elution time from 18.26 to 18.36 minutes is a peak of the compound (II), and a peak (3) for an elution time from 18.52 to 18.70 minutes is a peak of the compound (III).

### Experimental Example 1

### Hydroformylation Reaction

In a nitrogen atmosphere, 109.34 g of a DCPD-containing composition distillate 1 as a raw material compound for a hydroformylation reaction catalyst, 14.3 mg (0.0556 mmol) of Rh(acac)(CO)₂ as a catalyst, 1052 mg (1.63 mmol) of DBPO, and 84.28 g of methylcyclohexane as an organic solvent were charged in a stainless steel autoclave reactor having an internal capacity of 500 mL. Thereafter, while vertical stirring was performed, the temperature of a reaction liquid in the reactor was increased to 70°C. Subsequently, a gas mixture of hydrogen and carbon monoxide (hydrogen:carbon monoxide = 1:1 (molar ratio)) was injected rapidly through a gas introduction valve so that the pressure in the reactor reached 3 MPaG, and the reaction was performed until no hydrogen and no carbon monoxide were consumed by the reaction. Thereafter, while this pressure was maintained, the temperature of the reaction liquid was increased to 100°C, and the reaction was performed until no hydrogen and no carbon monoxide were consumed by the reaction. The time from the introduction of the gas mixture of hydrogen and carbon monoxide into the reactor until no hydrogen and no carbon monoxide were consumed by the reaction was defined as a reaction time of the hydroformylation reaction (also simply referred to as "reaction time" in the present specification), and used as an index of reaction efficiency. An amount of the gas mixture corresponding to that of the gas mixture consumed in the reaction during the reaction was automatically introduced into the autoclave through an automatic pressure regulation valve, and thus the reaction was performed while the pressure in the reactor was constantly maintained at 3 MPaG.

After completion of the reaction, the reaction liquid in the reactor was cooled to room temperature, and the remaining gas in the reactor was discharged, to produce 237.68 g of a hydroformylation reaction product liquid.

### Evaluation of Hydroformylation Reaction

For the produced hydroformylation reaction product liquid, by using a gas chromatograph (GC) and a method using total area in gas chromatography under the following GC measurement conditions, the content proportion (unit: GC area%) of dicyclopentadiene (DCPD) and the content proportions (unit: GC area%) of DCPD and the product tricyclodecane dicarbaldehyde (TCDDD) contained in the hydroformylation reaction product liquid were determined, and a DCPD conversion rate (unit: %) and a TCDDD yield (unit: %) were calculated. The DCPD conversion rate was 99.4%, and the TCDDD yield was 96.5%. The reaction time of the hydroformylation reaction was 4.88 hours. The evaluation results are shown in Table 2.

### GC Measurement Conditions

GC apparatus: gas chromatogram measuring apparatus (trade name: GC-2025, available from Shimadzu Corporation)
Detector: hydrogen flame ionization detector (FID)
Carrier gas: helium (column flow rate: 1.23 mL/min)
Column: capillary column (product name: DB-1, available from Agilent Technologies; size: length 30 m × inner diameter 0.25 mm, film thickness 1.00 µm)
Column temperature: 120°C (retention time: none) → temperature increase at 10°C/min → 300°C (retention time: 12 minutes)
Inlet temperature: 200°C
Detector temperature: 300°C
Sample amount: 0.3 µL (split ratio: 1/30)

### Extraction Operation

To 235.23 g of the produced hydroformylation reaction product liquid, 71.65 g of methanol and 46.85 g of water were added and then stirred in a nitrogen atmosphere for 40 minutes. Thereafter, the mixture was allowed to stand for 30 minutes to be separated into two phases, and a lower phase (a1) and an upper phase (b1) were separated and recovered.

To the resultant lower phase (a1), 8.4 g of methylcyclohexane was added and then stirred for 30 minutes. Thereafter, the mixture was allowed to stand for 30 minutes to be separated into two phases, and 278.83 g of a lower phase (a2) and 81.71 g of a mixed liquid of an upper phase (b1) and an upper phase (b2) were separated and recovered.

The composition of the resultant lower phase (a2) was analyzed by gas chromatography, and the lower phase was found to contain 47 mass% of tricyclodecane dicarbaldehyde, 27 mass% of methanol, 16 mass% of water, 7 mass% of methylcyclohexane, and 3 mass% of other components.

The recovery rate of rhodium (Rh) metal and the recovery rate of tris(2,4-di-tert-butylphenyl) phosphite in the mixed liquid of the upper phase (b1) and the upper phase (b2) were analyzed by fluorescent X-ray analysis and inductively coupled plasma emission spectrometry, and were 99% and 99%, respectively.

### Calculation of Reaction Rate Constant (k2) of Hydroformylation Reaction

A graph was drawn on an XY graph with the hydroformylation reaction time as the X-axis and the value of the natural logarithm of "1 - conversion rate/100" as the Y-axis under the assumption that the reaction rate of the hydroformylation reaction can be approximated by a linear function with respect to the content proportion of DCPD as a substrate. The slope of an approximate expression of the linear function in a range of the conversion rate from 60% to 80% was calculated, and this was defined as a reaction rate constant (k2) (unit: hr⁻¹) of the hydroformylation reaction.

The "conversion rate" was calculated by the same method as the method described in the evaluation of the hydroformylation reaction.

As a result, the reaction rate constant (k2) of the hydroformylation reaction in Experimental Example 1 was 0.98 hr⁻¹.

The evaluation results in Experimental Example 1 are summarized in Table 2.

### Experimental Example 2

The hydroformylation reaction of the raw material compound was performed under the same conditions as those in Experimental Example 1, except for using a DCPD-containing composition distillate 2 as the raw material compound in the hydroformylation reaction of Experimental Example 1, and evaluation was performed in the same manner.

The rate of decrease in the reaction rate constant (k2) in the hydroformylation reaction was calculated by the following method.

### Calculation of Rate of Decrease in Reaction Rate Constant (k2) of Hydroformylation Reaction

The reaction rate constant (k2) of the hydroformylation reaction calculated by the above-described method was divided by the reaction rate constant (k2) of Experimental Example 1, and the result was multiplied by 100 to provide the "rate of decrease in the reaction rate constant (k2) of the hydroformylation reaction".

The reaction rate constant (k2) of the hydroformylation reaction of Experimental Example 2 was 0.94 hr⁻¹, and the rate of decrease in the reaction rate constant (k2) was 4.1%. The evaluation results in Experimental Example 2 are summarized in Table 2.

### Experimental Example 3

The hydroformylation reaction of the raw material compound was performed under the same conditions as those in Experimental Example 1, except for using a DCPD-containing composition distillate 3 as the raw material compound in the hydroformylation reaction of Experimental Example 1, and evaluation was performed in the same manner. The rate of decrease in the reaction rate constant (k2) of the hydroformylation reaction was calculated in the same manner as in Experimental Example 2. The evaluation results are summarized in Table 2.

### Experimental Example 4

The hydroformylation reaction of the raw material compound was performed under the same conditions as those in Experimental Example 1, except for using a DCPD-containing composition distillate 4 as the raw material compound in the hydroformylation reaction of Experimental Example 1, and evaluation was performed in the same manner. The rate of decrease in the reaction rate constant (k2) of the hydroformylation reaction was calculated in the same manner as in Experimental Example 2. The evaluation results are summarized in Table 2.

### Experimental Example 5

The hydroformylation reaction of the raw material compound was performed under the same conditions as those in Experimental Example 1, except for using a DCPD-containing composition distillate 5 as the raw material compound in the hydroformylation reaction of Experimental Example 1, and evaluation was performed in the same manner. The rate of decrease in the reaction rate constant (k2) of the hydroformylation reaction was calculated in the same manner as in Experimental Example 2. The evaluation results are summarized in Table 2.

### Experimental Example 6

The hydroformylation reaction of the raw material compound was performed under the same conditions as those in Experimental Example 1, except for using a DCPD-containing composition distillate 6 as the raw material compound in the hydroformylation reaction of Experimental Example 1, and evaluation was performed in the same manner. The rate of decrease in the reaction rate constant (k2) of the hydroformylation reaction was calculated in the same manner as in Experimental Example 2. The evaluation results are summarized in Table 2.

### Experimental Example 7

The hydroformylation reaction of the raw material compound was performed under the same conditions as those in Experimental Example 1, except for using a DCPD-containing composition distillate 7 as the raw material compound in the hydroformylation reaction of Experimental Example 1, and evaluation was performed in the same manner. The rate of decrease in the reaction rate constant (k2) of the hydroformylation reaction was calculated in the same manner as in Experimental Example 2. The evaluation results are summarized in Table 2.

In Experimental Examples 1 to 7, the content proportions of the compound (I), the compound (II), and the compound (III) contained in extraction liquids 1 to 7 were measured according to measurement method 1 described above. The measurement results are shown in Table 2.

| | DCPD-containing composition | | | | | | | Reaction rate constant k2 | Rate of decrease in reaction rate constant k2 | Reaction time | Rh recovery rate |
|---|---|---|---|---|---|---|---|---|---|---|---|
| | Type | DCPD | DCPD oxide | | | | GC area ratio of DCPD oxide to DCPD | | | | |
| | | | Total | Compound (I) | Compound (II) | Compound (III) | | | | | |
| | | GC area% | GC area% | GC area% | GC area% | GC area% | - | hr⁻¹ | % | hr | % |
| Experimental Example 1 | Distillate 1 | 99.9 | 0.000 | 0.000 | 0.000 | 0.000 | 0.0000 | 0.98 | 0.0 | 4.88 | 99.0 |
| Experimental Example 2 | Distillate 2 | 98.7 | 0.007 | 0.003 | 0.000 | 0.004 | 0.0001 | 0.94 | 4.1 | 4.62 | 98.8 |
| Experimental Example 3 | Distillate 3 | 97.4 | 0.077 | 0.040 | 0.008 | 0.029 | 0.0008 | 0.86 | 12.2 | 4.83 | 95.8 |
| Experimental Example 4 | Distillate 4 | 99.6 | 0.150 | 0.048 | 0.014 | 0.088 | 0.0015 | 0.94 | 4.1 | 4.78 | 98.0 |
| Experimental Example 5 | Distillate 5 | 99.5 | 0.198 | 0.062 | 0.016 | 0.120 | 0.0020 | 0.88 | 10.2 | 4.85 | 94.8 |
| Experimental Example 6 | Distillate 6 | 98.1 | 0.304 | 0.100 | 0.018 | 0.186 | 0.0031 | 0.84 | 14.3 | 5.07 | 95.3 |
| Experimental Example 7 | Distillate 7 | 99.1 | 0.387 | 0.115 | 0.026 | 0.246 | 0.0039 | 0.81 | 17.3 | 6.32 | 87.8 |

FIG. 2 illustrates the relationship between the content proportion (GC area%) of the DCPD oxide in the DCPD-containing compositions (distillates 1 to 7) and the reaction rate constant (k2) of the hydroformylation reaction in Experimental Examples 1 to 7.

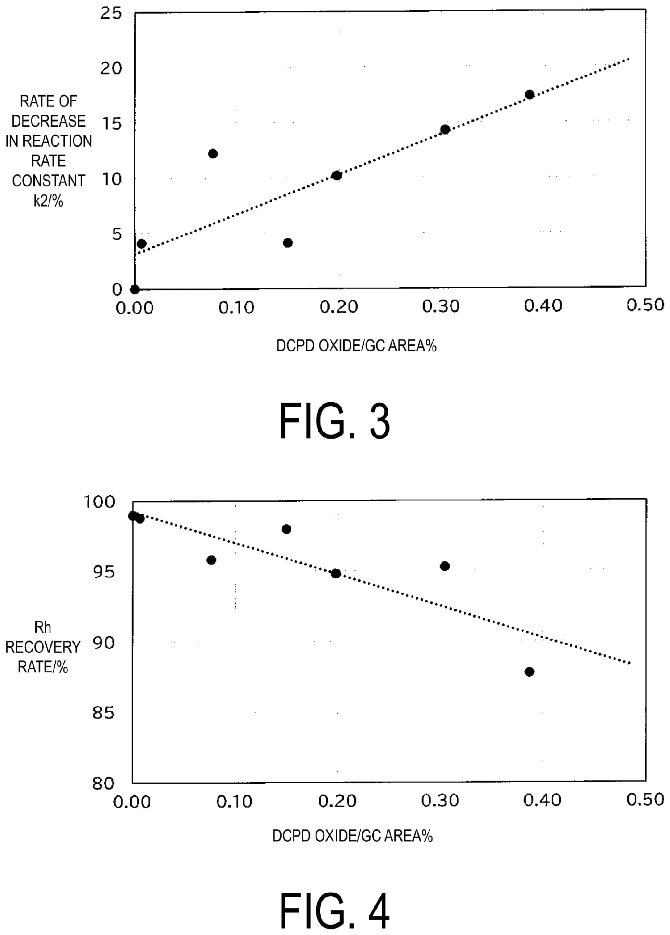

FIG. 3 illustrates the relationship between the content proportion (GC area%) of the DCPD oxide in the DCPD-containing compositions (distillates 1 to 7) and the rate of decrease in the reaction rate constant (k2) in Experimental Examples 1 to 7.

FIG. 4 illustrates the relationship between the content proportion (GC area%) of the DCPD oxide in the DCPD-containing composition (distillates 1 to 7) and the rhodium (Rh) recovery rate in Experimental Examples 1 to 7.

As is clear from FIG. 2, the lower the content proportion of the DCPD oxide in the DCPD-containing composition, the higher the value of the reaction rate constant (k2) of the hydroformylation reaction and the more excellent the reaction efficiency.

As is clear from FIG. 3, the lower the content proportion of the DCPD oxide in the DCPD-containing composition, the lower the value of the rate of decrease in the reaction rate constant (k2) of the hydroformylation reaction and the more excellent the reaction efficiency.

As is clear from FIG. 4, the lower the content proportion of the DCPD oxide in the DCPD-containing composition, the higher the rhodium (Rh) recovery rate.

### Experimental Example 8

### Production of Alcohol

102.1 g of the lower phase (a2) obtained in Experimental Example 1 and 2 g of a nickel-chromium-supported diatomaceous earth catalyst as a hydrogenation catalyst were charged in an autoclave reactor having an internal capacity of 200 L. Thereafter, while stirring was performed at 1200 rpm, the temperature of a reaction liquid in the reactor was increased to 160°C. Subsequently, hydrogen gas was injected through a gas introduction valve to set the pressure in the reactor to 3 MPaG and, while this pressure and the temperature of the reaction liquid were maintained, the reaction was allowed to proceed for 2 hours. An amount of the hydrogen gas corresponding to that consumed in the reaction during the reaction was continuously introduced into the reactor while the pressure in the reactor was maintained at 3 MPaG.

After completion of the reaction, the reaction liquid in the reactor was cooled to room temperature, the remaining gas in the reactor was discharged, and the nickel-chromium-supported diatomaceous earth catalyst was separated by filtration using a 5-µm filter, to produce 97.2 g of a reaction product liquid. The content of tricyclodecane dicarbaldehyde as the raw material compound contained in the reaction liquid before the reaction and the content of tricyclo[5.2.1.0(2,6)]decanedimethanol (hereinafter, referred to as "TCDDM") as a product in the reaction product liquid after the reaction were analyzed by gas chromatography. As a result, the TCDDM yield was 97.9%.

### Reference Experimental Example 3

### Distillation Purification of Dicyclopentadiene (DCPD)

In a single distillation column made of glass, 1912 g of DCPD was charged, and distillation was performed under conditions of a pressure of 0.1 kPa, a column bottom temperature of 65°C, and a column top temperature of 60°C to produce 1832 g of a distillate. The distillate was used as a DCPD-containing composition. The content proportion of DCPD in the DCPD-containing composition was 99.7 GC area%.

The peroxide concentration in the DCPD-containing composition was measured by an iodometric titration method according to the following procedure.

In a nitrogen atmosphere, 5 mL of DCPD was added to a 50-mL pear-shaped flask, then 7.5 mL of a mixed liquid of chloroform and acetic acid (acetic acid:chloroform = 3:2, volume ratio) was added, and then 0.2 mL of a saturated aqueous potassium iodide solution was further added, followed by stirring until the solution in the flask turned yellow or brown. Further, 12.5 mL of distilled water was added for dilution, followed by stirring for 1 minute, and the resultant product was used as a measurement sample. While the measurement sample was stirred, an aqueous sodium thiosulfate solution (0.01 mol/L) as a standard liquid was added dropwise until the measurement sample became colorless and transparent, and iodometric titration was performed. The peroxide concentration (unit: mmol/L) in the DCPD-containing composition was calculated based on the consumed amount of the dropped aqueous sodium thiosulfate solution, and, as a result, the peroxide concentration was 0.031 mmol/L.

### Reference Experimental Example 4

### Hydroformylation Reaction (First)

In a nitrogen atmosphere, 112.9 g of a DCPD-containing composition produced in Reference Experimental Example 3 as a raw material compound for a hydroformylation reaction catalyst, 13.4 mg (0.0519 mmol) of Rh(acac)(CO)₂ as a catalyst, 1052.8 mg (1.623 mmol) of DBPO, and 85.4 g of methylcyclohexane as an organic solvent were charged in a stainless steel autoclave reactor having an internal capacity of 100 mL. Thereafter, while stirring was performed at 1500 rpm, the temperature of a reaction liquid in the reactor was increased to 70°C. Subsequently, a gas mixture of hydrogen and carbon monoxide (hydrogen:carbon monoxide = 1:1 (molar ratio)) was injected rapidly through a gas introduction valve so that the pressure in the reactor reached 3 MPaG. While this pressure was maintained, the temperature of the reaction liquid was increased to 100°C, and the reaction was performed until no hydrogen and no carbon monoxide were consumed by the reaction. The time from the introduction of the gas mixture of hydrogen and carbon monoxide into the reactor until no hydrogen and no carbon monoxide were consumed by the reaction was defined as a reaction time of the hydroformylation reaction (also simply referred to as "reaction time" in the present specification), and used as an index of reaction efficiency. An amount of the gas mixture corresponding to that of the gas mixture consumed in the reaction during the reaction was automatically introduced into the autoclave through an automatic pressure regulation valve, and thus the reaction was performed while the pressure in the reactor was constantly maintained at 3 MPaG.

After completion of the reaction, the reaction liquid in the reactor was cooled to room temperature, and the remaining gas in the reactor was discharged, to produce 244.6 g of a hydroformylation reaction product liquid.

### Evaluation of Hydroformylation Reaction (First)

For the DCPD-containing composition produced in Reference Experimental Example 3 and the hydroformylation reaction product liquid produced in Reference Experimental Example 4, by using a gas chromatograph (GC) and a method using total area in gas chromatography under the following GC measurement conditions, the content proportions (unit: GC area%) of DCPD and the product tricyclodecane dicarbaldehyde (TCDDD) contained in the hydroformylation reaction product liquid were determined using the DCPD content proportion (99.7 GC area%) of the DCPD-containing composition produced in Reference Experimental Example 3, and a DCPD conversion rate (unit: %) and a TCDDD yield (unit: %) were calculated. The DCPD conversion rate was 99.4%, and the TCDDD yield was 96.5%. The reaction time of the hydroformylation reaction was 5.21 hours.

### GC Measurement Conditions

GC apparatus: GC-2025 (high-performance general-purpose gas chromatograph, available from Shimadzu Corporation)
Detector: hydrogen flame ionization detector (FID)
Carrier gas: helium (column flow rate: 1.23 mL/min)
Column: Capillary column DB-1 (available from Agilent Technologies; size: length 30 m × inner diameter 0.25 mm, film thickness 1.00 µm)
Column temperature: 120°C (retention time: none) → temperature increase at 10°C/min → 300°C (retention time: 12 minutes)
Inlet temperature: 200°C
Detector temperature: 300°C
Sample amount: 0.3 µL (split ratio: 1/30)

### Extraction Operation

To 224.6 g of the hydroformylation reaction product liquid produced in Reference Experimental Example 4, 74.04 g of methanol and 47.7 g of water were added and then stirred in a nitrogen atmosphere for 30 minutes. Thereafter, the mixture was allowed to stand for 30 minutes to be separated into two phases, and a lower phase (a1) and an upper phase (b1) were separated and recovered.

To the resultant lower phase (a1), 9.6 g of methylcyclohexane was added and then stirred for 40 minutes. Thereafter, the mixture was allowed to stand for 30 minutes to be separated into two phases, and 287.4 g of a lower phase (a2) and 84.01 g of a mixed liquid of an upper phase (b1) and an upper phase (b2) were separated and recovered.

The composition of the resultant lower phase (a2) was analyzed by gas chromatography, and the lower phase was found to contain 47 mass% of tricyclodecane dicarbaldehyde, 27 mass% of methanol, 16 mass% of water, 7 mass% of methylcyclohexane, and 3 mass% of other components.

The recovery rate of rhodium (Rh) metal and the recovery rate of tris(2,4-di-tert-butylphenyl)phosphite in the obtained mixed liquid of the upper phase (b1) and the upper phase (b2) were analyzed by fluorescent X-ray analysis and inductively coupled plasma emission spectrometry, and were 99.0% and 99.0%, respectively.

### Experimental Example 9

### Hydroformylation Reaction (Second to Fourth)

The hydroformylation reaction and the extraction operation were performed in the same manner as in Reference Experimental Example 4, except that the mixed liquid of the upper phases (b1) and (b2) obtained by the extraction operation of Reference Experimental Example 4 was used instead of the catalyst and solvent for the hydroformylation reaction in Reference Experimental Example 4, and that rhodium (Rh) metal and DBPO in amounts lost by the extraction operation of Reference Experimental Example 4 were added to the mixed liquid so as to achieve contents equivalent to the contents of Rh metal and DBPO in the first hydroformylation reaction of Reference Experimental Example 4. This operation is referred to as the "second" hydroformylation reaction operation.

The above operation was repeated twice to produce reaction liquids of the second to fourth hydroformylation reactions.

The reaction liquids of the second to fourth hydroformylation reactions were analyzed using the gas chromatograph (GC) and method using total area in gas chromatography. A difference in reaction time between the second and fourth hydroformylation reactions (hereinafter, also referred to as "reaction time difference") was 1 minute.

The rate of decrease in the reaction rate constant per hydroformylation reaction in the second to fourth hydroformylation reactions, as measured by measurement method 2 below, was 0.5%.

### Measurement Method 2

### Rate of Decrease in Reaction Rate Constant per Hydroformylation Reaction

Procedure 1) Measurement of Reaction Rate Constant of Hydroformylation Reaction:
A graph is drawn on an XY graph with the hydroformylation reaction time as the X-axis and the value of the natural logarithm of "1 - conversion rate/100" as the Y-axis under the assumption that the reaction rate of the hydroformylation reaction can be approximated by a linear function with respect to the concentration of DCPD as a substrate. The slope of an approximate expression of the linear function in a range of the conversion rate from 60% to 80% is calculated, and is defined as the reaction rate constant of the hydroformylation reaction.

The "conversion rate" is measured by the same method as the method described in the evaluation of the hydroformylation reaction (first).

Procedure 2) The reaction rate constants in the second to fourth hydroformylation reactions are plotted on an XY graph with the number of hydroformylation reactions as the X axis and the reaction rate constant of the hydroformylation reaction calculated in the procedure 1 as the Y axis; a slope when approximated by a linear function is calculated; and a result of dividing by the reaction rate constant of the second hydroformylation reaction and multiplying by 100 is defined as the "rate of decrease in the reaction rate constant per hydroformylation reaction in the second to fourth hydroformylation reactions".

### Experimental Example 10

The DCPD-containing composition produced in Reference Experimental Example 3 was charged in a 1-L glass bottle. Then, in order to suppress the formation of a dicyclopentadiene-derived peroxide due to the oxidation of dicyclopentadiene, 2,6-di-tert-butyl-4-hydroxytoluene (BHT) was added so as to achieve a concentration of 150 ppm by mass, and the glass bottle was closed with a lid and then stored for 1 month.

The peroxide concentration in the DCPD-containing composition after storage for 1 month was measured by the same method as in Experimental Example 9, and was found to be 0.323 mmol/L.

Further, the hydroformylation reaction and the extraction operation were repeated three times in the same manner as in Experimental Example 9, and the difference in reaction time between the second and fourth hydroformylation reactions was 3 minutes. The rate of decrease in the reaction rate constant per hydroformylation reaction in the second to fourth hydroformylation reactions was 1.2%.

### Comparative Experimental Example 1

The DCPD-containing composition produced in Reference Experimental Example 3 was charged in a 1-L glass bottle, and the glass bottle was closed with a lid and then stored at room temperature (25°C) for 1 month.

The peroxide concentration in the DCPD-containing composition after storage for 1 month was measured by the same method as in Experimental Example 9, and was found to be 2.933 mmol/L.

Further, the hydroformylation reaction and the extraction operation were repeated three times in the same manner as in Experimental Example 9, and the difference in reaction time between the second and fourth hydroformylation reactions was 23 minutes. In addition, the rate of decrease in the reaction rate constant per hydroformylation reaction in the second to fourth hydroformylation reactions was 7.4%.

The results of Experimental Examples 9 and 10 and Comparative Experimental Example 1 are summarized in Table 3 below.

**[Table 3]**

| | Content proportion of DCPD in DCPD-containing composition | Concentration of peroxide in DCPD-containing composition | Rate of decrease in reaction rate constant per hydroformylation reaction in second to fourth hydroformylation reactions | Difference in reaction time between second hydroformylation reaction and fourth hydroformylation reaction |
|---|---|---|---|---|
| Unit | GC area% | mmol/L | % | Time (min) |
| Experimental Example 9 | 99.7 | 0.031 | 0.5 | 1 |
| Experimental Example 10 | 99.7 | 0.323 | 1.2 | 3 |
| Comparative Experimental Example 1 | 99.7 | 2.933 | 7.4 | 23 |

In Experimental Examples 9 and 10, the time difference between the second and fourth hydroformylation reactions was small.

The rate of decrease in the reaction rate constant per hydroformylation reaction in the second to fourth hydroformylation reactions was also small.

In contrast, in Comparative Experimental Example 1, the peroxide concentration in the cyclic diene-containing composition was higher than that in Experimental Examples 9 and 10. Therefore, the time difference between the second and fourth hydroformylation reactions was large, and the rate of decrease in the reaction rate constant per hydroformylation reaction in the second to fourth hydroformylation reactions was also large. This indicates that when the peroxide concentration in the cyclic diene-containing composition is high, the activity of the rhodium-organic ligand complex catalyst contained in the reaction product liquid after the hydroformylation reaction is impaired by the peroxide due to repetition of the hydroformylation reaction.

From the above results, it is found that by controlling the peroxide concentration in the cyclic diene-containing composition, even when a procedure including recovery of the rhodium-organic ligand complex catalyst contained in the reaction product liquid after the hydroformylation reaction and reuse is repeated, the activity of the catalyst can be prevented from being lowered, and the hydroformylation reaction efficiency can be maintained well.

### Experimental Example 11

### Production of Alcohol

106.1 g of the lower phase (a2) obtained in Reference Experimental Example 3 and 2 g of a nickel-chromium-supported diatomaceous earth catalyst were charged in an autoclave reactor having an internal capacity of 0.2 L. Thereafter, while stirring was performed at 120 rpm, the temperature of a reaction liquid in the reactor was increased to 160°C. Subsequently, hydrogen gas was injected into the reactor through a gas introduction valve to set the pressure in the reactor to 3 MPaG and, while this pressure and the temperature of the reaction liquid were maintained, the reaction was allowed to proceed for 2.5 hours. An amount of the hydrogen gas corresponding to that consumed in the reaction during the reaction was continuously introduced into the reactor while the pressure in the reactor was maintained at 3 MPaG.

After completion of the reaction, the reaction liquid in the reactor was cooled to room temperature, the remaining gas in the reactor was discharged, and the nickel-chromium-supported diatomaceous earth catalyst was separated by filtration using a 5-µm filter, to produce 98.3 g of a reaction product liquid. The content of tricyclodecane dicarbaldehyde as the raw material compound contained in the reaction liquid before the reaction and the content of tricyclo[5.2.1.0^{2,6}]decanedimethanol (hereinafter, referred to as "TCDDM") as a product in the reaction product liquid after the reaction were analyzed by gas chromatography. As a result, the TCDDM yield was 98%.

Although the present invention has been described in detail with reference to specific embodiments, it will be apparent to those skilled in the art that various modifications can be made within the scope where the effects of the present invention are achieved.

The present application is based on JP 2024-015094 filed on February 2, 2024, and JP 2024-018008 filed on February 8, 2024, which are incorporated herein by reference in their entirety.

### Reference Signs List

1 Dimerization tank
2 Distillation column
3 Low-boiling-point component removal column
4 High-boiling-point component removal column

## Claims

1. A method for producing an aldehyde by subjecting a cyclic diene in a cyclic diene-containing composition to a hydroformylation reaction,
the method comprising setting a content proportion of an oxygen-containing compound contained in the cyclic diene-containing composition to a predetermined threshold or less.

2. The method for producing an aldehyde according to claim 1, wherein the oxygen-containing compound is a cyclic diene oxide, and the cyclic diene excludes the cyclic diene oxide.

3. The method for producing an aldehyde according to claim 2, wherein a content proportion of the cyclic diene oxide contained in the cyclic diene-containing composition is 1.2 GC area% or less.

4. The method for producing an aldehyde according to claim 2, wherein a content proportion of the cyclic diene contained in the cyclic diene-containing composition is 60 GC area% or more.

5. The method for producing an aldehyde according to claim 3, wherein the content proportion of the cyclic diene oxide contained in the cyclic diene-containing composition is 0.5 GC area% or less, preferably 0.35 GC area% or less, more preferably 0.25 GC area% or less, further preferably 0.14 GC area% or less, and particularly preferably 0.05 GC area% or less.

6. The method for producing an aldehyde according to claim 2, wherein a content proportion of the cyclic diene oxide is measured by measurement method 1 described below:
measurement method 1
measuring a total content proportion of peaks for an elution time from 17.7 minutes to 18.8 minutes by analysis under GC measurement conditions described below:
GC measurement conditions
GC apparatus: gas chromatogram measuring apparatus (trade name: GC-2025, available from Shimadzu Corporation)
detector: hydrogen flame ionization detector (FID)
carrier gas: helium (column flow rate: 1.65 mL/min)
column: capillary column (product name: DB-1, available from Agilent Technologies; size: length 30 m × inner diameter 0.25 mm, film thickness 1.00 µm)
column temperature: 50°C (retention time: 5 minutes) → temperature increase at 10°C/min → 300°C (retention time: none)
inlet temperature: 200°C
detector temperature: 300°C
sample amount: 0.3 µL (split ratio: 1/30).

7. The method for producing an aldehyde according to claim 2, wherein the cyclic diene is a polycyclic diene.

8. The method for producing an aldehyde according to claim 7, wherein the cyclic diene is dicyclopentadiene.

9. The method for producing an aldehyde according to claim 2, wherein the cyclic diene oxide is a compound having any skeleton selected from a tricyclodecene skeleton and a tricyclodecadiene skeleton, and having an oxygen-containing functional group.

10. The method for producing an aldehyde according to claim 2, wherein the cyclic diene oxide contains at least one selected from the group consisting of a cyclic diene having a carbonyl group, a cyclic diene having a hydroxyl group, and a cyclic monoene having an epoxy group, which correspond to the cyclic diene.

11. The method for producing an aldehyde according to claim 8, wherein
the cyclic diene is dicyclopentadiene, and
the cyclic diene oxide contains at least one selected from the group consisting of a compound in which one enantiomer is represented by General Formula (I) described below, a compound in which one enantiomer is represented by General Formula (II) described below, and a compound in which one enantiomer is represented by General Formula (III) described below: 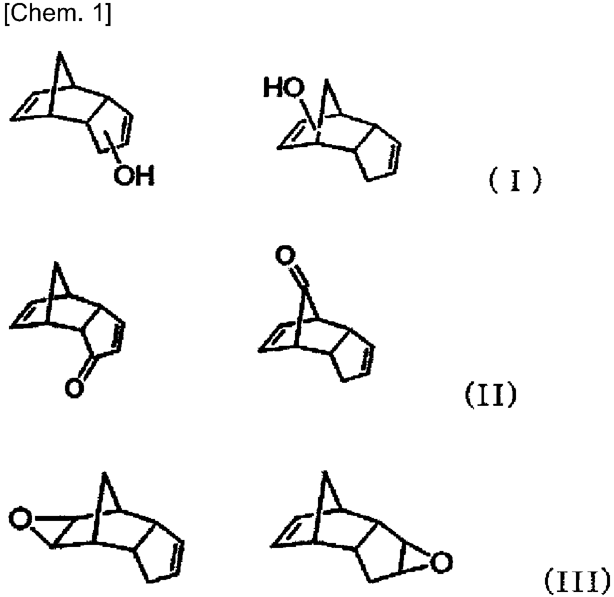
where, in General Formula (I), a hydroxyl group (-OH) is bonded to any position in an illustrated dicyclopentadiene ring structure.

12. The method for producing an aldehyde according to claim 11, wherein the cyclic diene is dicyclopentadiene, and the aldehyde is tricyclodecane dicarbaldehyde.

13. The method for producing an aldehyde according to claim 2, wherein
the cyclic diene-containing composition is a composition obtained by thermal decomposition of a hydrocarbon-containing composition to produce a hydrocarbon decomposition product, and distillation purification of the hydrocarbon decomposition product, and
the method comprises performing the distillation purification so that a content proportion of the cyclic diene oxide contained in the cyclic diene-containing composition is a predetermined threshold or less.

14. The method for producing an aldehyde according to claim 2, the method comprising controlling a storage condition of the cyclic diene-containing composition so that a content proportion of the cyclic diene oxide contained in the cyclic diene-containing composition is a predetermined threshold or less.

15. The method for producing an aldehyde according to claim 14, wherein the controlling the storage condition includes controlling a dissolved oxygen concentration in the cyclic diene-containing composition.

16. The method for producing an aldehyde according to claim 14, wherein the controlling the storage condition includes adding an antioxidant to the cyclic diene-containing composition.

17. A method for producing an alcohol, the method comprising producing an aldehyde by the method described in any one of claims 1 to 16, and producing an alcohol from the aldehyde.

18. The method for producing an alcohol according to claim 17, wherein the cyclic diene is dicyclopentadiene, the aldehyde is tricyclodecane dicarbaldehyde, and the alcohol is tricyclo[5.2.1.0^{2,6}]decanedimethanol.

19. The method for producing an aldehyde according to claim 1, wherein the oxygen-containing compound is a peroxide.

20. The method for producing an aldehyde according to claim 19, wherein a concentration of the peroxide contained in the cyclic diene-containing composition is 2.8 mmol/L or less.

21. The method for producing an aldehyde according to claim 20, wherein the concentration of the peroxide contained in the cyclic diene-containing composition is 2.0 mmol/L or less, preferably 1.0 mmol/L or less, more preferably 0.6 mmol/L or less, and particularly preferably 0.3 mmol/L or less.

22. The method for producing an aldehyde according to claim 19, wherein the peroxide contains a peroxide of an alkene compound.

23. The method for producing an aldehyde according to claim 19, wherein a concentration of the peroxide is measured by measurement method 1 described below:
measurement method 1
adding a mixed liquid of chloroform and acetic acid to a cyclic diene-containing composition under a nitrogen atmosphere, and further adding a saturated aqueous potassium iodide solution, followed by stirring and dilution with distilled water to prepare a measurement sample; and subjecting the prepared measurement sample to iodometric titration using a 0.01 mol/L aqueous sodium thiosulfate solution as a standard liquid, and determining the concentration (mmol/L) of the peroxide in the cyclic diene-containing composition based on an amount of the standard liquid consumed.

24. The method for producing an aldehyde according to claim 19, wherein the cyclic diene is a polycyclic diene.

25. The method for producing an aldehyde according to claim 24, wherein the polycyclic diene is dicyclopentadiene.

26. The method for producing an aldehyde according to claim 19, wherein
the cyclic diene-containing composition is a composition obtained by thermal decomposition of a hydrocarbon-containing composition to produce a hydrocarbon decomposition product, and distillation purification of the hydrocarbon decomposition product, and
the method comprises performing the distillation purification so that a concentration of the peroxide contained in the cyclic diene-containing composition is a predetermined threshold or less.

27. The method for producing an aldehyde according to claim 26, wherein the hydrocarbon decomposition product contains an antioxidant.

28. The method for producing an aldehyde according to claim 26, the method comprising adding an antioxidant to the hydrocarbon decomposition product so that the concentration of the peroxide contained in the cyclic diene-containing composition is a predetermined threshold or less.

29. The method for producing an aldehyde according to claim 26, the method comprising controlling a storage condition of the cyclic diene-containing composition so that the concentration of the peroxide contained in the cyclic diene-containing composition is a predetermined threshold or less.

30. The method for producing an aldehyde according to claim 29, wherein the controlling the storage condition includes controlling a dissolved oxygen concentration in the cyclic diene-containing composition.

31. The method for producing an aldehyde according to claim 29, wherein the controlling the storage condition includes adding an antioxidant to the cyclic diene-containing composition.

32. The method for producing an aldehyde according to claim 19, wherein the cyclic diene is dicyclopentadiene, and the aldehyde is tricyclodecane dicarbaldehyde.

33. A method for producing an alcohol, the method comprising producing an aldehyde by the method described in any one of claims 19 to 32, and producing a corresponding alcohol from the produced aldehyde.

34. The method for producing an alcohol according to claim 33, wherein the cyclic diene is dicyclopentadiene, the aldehyde is tricyclodecane dicarbaldehyde, and the alcohol is tricyclo[5.2.1.0^{2,6}]decanedimethanol.

35. A method for producing a cyclic diene-containing composition containing a cyclic diene by thermal decomposition of a hydrocarbon-containing composition to produce a hydrocarbon decomposition product, and distillation purification of the hydrocarbon decomposition product,
the method comprising setting a content proportion of an oxygen-containing compound contained in the cyclic diene-containing composition to a predetermined threshold or less.

36. The method for producing a cyclic diene-containing composition according to claim 35, wherein the oxygen-containing compound is a cyclic diene oxide, and the cyclic diene excludes the cyclic diene oxide.

37. The method for producing a cyclic diene-containing composition according to claim 36, wherein a content proportion of the cyclic diene oxide contained in the cyclic diene-containing composition is 1.2 GC area% or less.

38. The method for producing a cyclic diene-containing composition according to claim 36, the method comprising performing the distillation purification so that a content proportion of the cyclic diene oxide contained in the cyclic diene-containing composition is a predetermined threshold or less.

39. The method for producing a cyclic diene-containing composition according to claim 36, the method comprising controlling a storage condition of the cyclic diene-containing composition so that a content proportion of the cyclic diene oxide contained in the cyclic diene-containing composition is a predetermined threshold or less.

40. The method for producing a cyclic diene-containing composition according to claim 39, wherein the controlling the storage condition includes controlling a dissolved oxygen concentration in the cyclic diene-containing composition.

41. The method for producing a cyclic diene-containing composition according to claim 39, wherein the controlling the storage condition includes adding an antioxidant to the cyclic diene-containing composition.

42. The method for producing a cyclic diene-containing composition according to claim 36, wherein a content proportion of the cyclic diene contained in the cyclic diene-containing composition is 60 GC area% or more.

43. The method for producing a cyclic diene-containing composition according to claim 41, wherein a content proportion of the cyclic diene oxide contained in the cyclic diene-containing composition is 0.5 GC area% or less, preferably 0.35 GC area% or less, more preferably 0.25 GC area% or less, further preferably 0.14 GC area% or less, and particularly preferably 0.05 GC area% or less.

44. The method for producing a cyclic diene-containing composition according to claim 36, wherein a content proportion of the cyclic diene oxide is measured by measurement method 1 described below:
measurement method 1
measuring a total content proportion of peaks for an elution time from 17.7 minutes to 18.8 minutes by analysis under GC measurement conditions described below:
GC measurement conditions
GC apparatus: gas chromatogram measuring apparatus (trade name: GC-2025, available from Shimadzu Corporation)
detector: hydrogen flame ionization detector (FID)
carrier gas: helium (column flow rate: 1.65 mL/min)
column: capillary column (product name: DB-1, available from Agilent Technologies; size: length 30 m × inner diameter 0.25 mm, film thickness 1.00 µm)
column temperature: 50°C (retention time: 5 minutes) → temperature increase at 10°C/min → 300°C (retention time: none)
inlet temperature: 200°C
detector temperature: 300°C
sample amount: 0.3 µL (split ratio: 1/30).

45. The method for producing a cyclic diene-containing composition according to claim 36, wherein the cyclic diene is a polycyclic diene.

46. The method for producing a cyclic diene-containing composition according to claim 45, wherein the cyclic diene is dicyclopentadiene.

47. The method for producing a cyclic diene-containing composition according to claim 36, wherein the cyclic diene oxide is a compound having any skeleton selected from a tricyclodecene skeleton and a tricyclodecadiene skeleton, and having an oxygen-containing functional group.

48. The method for producing a cyclic diene-containing composition according to claim 36, wherein the cyclic diene oxide contains at least one selected from the group consisting of a cyclic diene having a carbonyl group, a cyclic diene having a hydroxyl group, and a cyclic monoene having an epoxy group.

49. The method for producing a cyclic diene-containing composition according to claim 46, wherein
the cyclic diene is dicyclopentadiene, and
the cyclic diene oxide contains at least one selected from the group consisting of a compound in which one enantiomer is represented by General Formula (I) described below, a compound in which one enantiomer is represented by General Formula (II) described below, and a compound in which one enantiomer is represented by General Formula (III) described below: 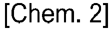 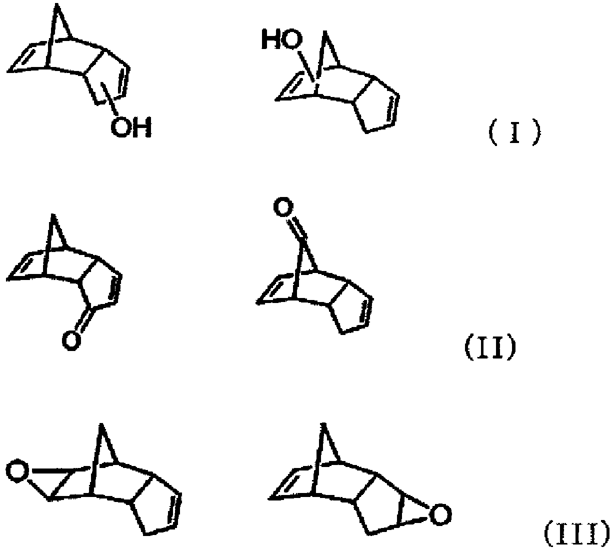
where, in General Formula (I), a hydroxyl group (-OH) is bonded to any position in an illustrated dicyclopentadiene ring structure.

50. The method for producing a cyclic diene-containing composition according to claim 36, wherein the hydrocarbon-containing composition is naphtha.

51. The method for producing a cyclic diene-containing composition according to claim 35, wherein the oxygen-containing compound is a peroxide.

52. The method for producing a cyclic diene-containing composition according to claim 51, the method comprising performing the distillation purification so that a concentration of the peroxide contained in the cyclic diene-containing composition is a predetermined threshold or less.

53. The method for producing a cyclic diene-containing composition according to claim 51, wherein the hydrocarbon decomposition product contains an antioxidant.

54. The method for producing a cyclic diene-containing composition according to claim 51, the method comprising adding an antioxidant to the hydrocarbon decomposition product so that a concentration of the peroxide contained in the cyclic diene-containing composition is a predetermined threshold or less.

55. The method for producing a cyclic diene-containing composition according to claim 51, the method comprising controlling a storage condition of the cyclic diene-containing composition so that a concentration of the peroxide contained in the cyclic diene-containing composition is a predetermined threshold or less.

56. The method for producing a cyclic diene-containing composition according to claim 55, wherein the controlling the storage condition includes controlling a dissolved oxygen concentration in the cyclic diene-containing composition.

57. The method for producing a cyclic diene-containing composition according to claim 55, wherein the controlling the storage condition includes adding an antioxidant to the cyclic diene-containing composition.

58. The method for producing a cyclic diene-containing composition according to claim 51, wherein a concentration of the peroxide contained in the cyclic diene-containing composition is 2.8 mmol/L or less.

59. The method for producing a cyclic diene-containing composition according to claim 51, wherein a content proportion of the cyclic diene contained in the cyclic diene-containing composition is 60.0 GC area% or more.

60. The method for producing a cyclic diene-containing composition according to claim 58, wherein the concentration of the peroxide contained in the cyclic diene-containing composition is 2.0 mmol/L or less, preferably 1.0 mmol/L or less, more preferably 0.6 mmol/L or less, and particularly preferably 0.3 mmol/L or less.

61. The method for producing a cyclic diene-containing composition according to claim 51, wherein a concentration of the peroxide is measured by measurement method 1 described below:
measurement method 1
adding a mixed liquid of chloroform and acetic acid to a cyclic diene-containing composition under a nitrogen atmosphere, and further adding a saturated aqueous potassium iodide solution, followed by stirring and dilution with distilled water to prepare a measurement sample; and subjecting the prepared measurement sample to iodometric titration using a 0.01 mol/L aqueous sodium thiosulfate solution as a standard liquid, and determining the concentration (mmol/L) of the peroxide in the cyclic diene-containing composition based on an amount of the standard liquid consumed.

62. The method for producing a cyclic diene-containing composition according to claim 51, wherein the cyclic diene is a polycyclic diene.

63. The method for producing a cyclic diene-containing composition according to claim 62, wherein the polycyclic diene is dicyclopentadiene.

64. The method for producing a cyclic diene-containing composition according to claim 51, wherein the hydrocarbon-containing composition is naphtha.

65. A cyclic diene-containing composition comprising a cyclic diene, wherein
a content proportion of a cyclic diene oxide in the cyclic diene-containing composition is 1.2 GC area% or less, and
the cyclic diene excludes the cyclic diene oxide.

66. The cyclic diene-containing composition according to claim 65, wherein a content proportion of the cyclic diene is 60 GC area% or more.

67. The cyclic diene-containing composition according to claim 65, wherein the content proportion of the cyclic diene oxide is 0.5 GC area% or less, preferably 0.35 GC area% or less, more preferably 0.25 GC area% or less, further preferably 0.14 GC area% or less, and particularly preferably 0.05 GC area% or less.

68. The cyclic diene-containing composition according to claim 65, wherein the content proportion of the cyclic diene oxide is measured by measurement method 1 described below:
measurement method 1
measuring a total content proportion of peaks for an elution time from 17.7 minutes to 18.8 minutes by analysis under GC measurement conditions described below:
GC measurement conditions
GC apparatus: gas chromatogram measuring apparatus (trade name: GC-2025, available from Shimadzu Corporation)
detector: hydrogen flame ionization detector (FID)
carrier gas: helium (column flow rate: 1.65 mL/min)
column: capillary column (product name: DB-1, available from Agilent Technologies; size: length 30 m × inner diameter 0.25 mm, film thickness 1.00 µm)
column temperature: 50°C (retention time: 5 minutes) → temperature increase at 10°C/min → 300°C (retention time: none)
inlet temperature: 200°C
detector temperature: 300°C
sample amount: 0.3 µL (split ratio: 1/30).

69. The cyclic diene-containing composition according to claim 65, wherein the cyclic diene is a polycyclic diene.

70. The cyclic diene-containing composition according to claim 69, wherein the cyclic diene is dicyclopentadiene.

71. The cyclic diene-containing composition according to claim 65, wherein the cyclic diene oxide is a compound having any skeleton selected from a tricyclodecene skeleton and a tricyclodecadiene skeleton, and having an oxygen-containing functional group.

72. The cyclic diene-containing composition according to claim 65, wherein the cyclic diene oxide contains at least one selected from the group consisting of a cyclic diene having a carbonyl group, a cyclic diene having a hydroxyl group, and a cyclic monoene having an epoxy group.

73. The cyclic diene-containing composition according to claim 70, wherein
the cyclic diene is dicyclopentadiene, and
the cyclic diene oxide contains at least one selected from the group consisting of a compound in which one enantiomer is represented by General Formula (I) described below, a compound in which one enantiomer is represented by General Formula (II) described below, and a compound in which one enantiomer is represented by General Formula (III) described below: 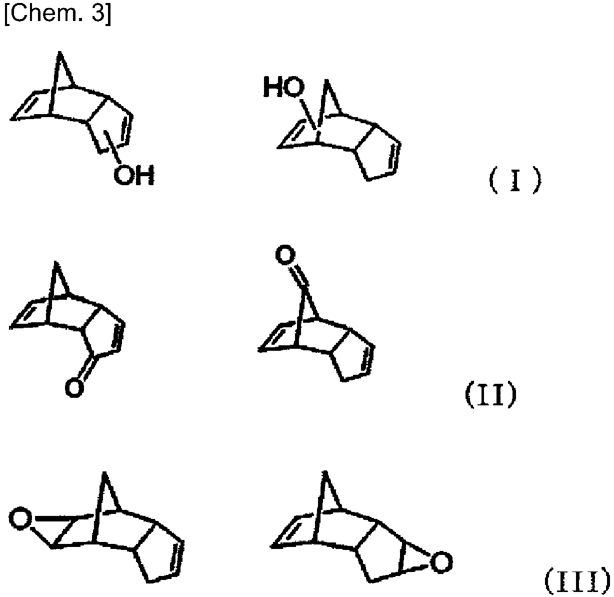
where, in General Formula (I), a hydroxyl group (-OH) is bonded to any position in an illustrated dicyclopentadiene ring structure.

74. The cyclic diene-containing composition according to claim 65, wherein a concentration of the peroxide in the cyclic diene-containing composition is 2.8 mmol/L or less.

75. The cyclic diene-containing composition according to claim 74, wherein the concentration of the peroxide is 2.0 mmol/L or less, preferably 1.0 mmol/L or less, more preferably 0.6 mmol/L or less, and particularly preferably 0.3 mmol/L or less.

76. The cyclic diene-containing composition according to claim 74, wherein the peroxide contains a peroxide of an alkene compound.

77. A method for producing an aldehyde by subjecting the cyclic diene-containing composition described in any one of claims 65 to 76 to a hydroformylation reaction.

78. A method for producing an alcohol, wherein an aldehyde is produced by the method described in claim 77, and an alcohol is produced from the aldehyde.
